(19) Europäisches Patentamt
European Patent Office
Office européen des brevets

(11) EP 4 782 013 A1

(12) EUROPEAN PATENT APPLICATION
published in accordance with Art. 153(4) EPC

(43) Date of publication:
**29.07.2026 Bulletin 2026/31**

(21) Application number: **24867550.6**

(22) Date of filing: **20.09.2024**

(51) International Patent Classification (IPC):
***A61K 47/68*** (2017.01) ***A61P 35/00*** (2006.01)
***A61K 39/395*** (2006.01)

(52) Cooperative Patent Classification (CPC):
**A61K 39/395; A61K 47/68; A61P 35/00**

(86) International application number:
**PCT/CN2024/120021**

(87) International publication number:
**WO 2025/061145 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.09.2023 CN 202311230811**
**29.05.2024 CN 202410679328**

(71) Applicant: **Jiangsu Hengrui Pharmaceuticals Co., Ltd.**
**Lianyungang, Jiangsu 222047 (CN)**

(72) Inventor: **SHI, Wei**
**Lianyungang, Jiangsu 222047 (CN)**

(74) Representative: **Regimbeau**
**20, rue de Chazelles**
**75847 Paris Cedex 17 (FR)**

Remarks:
The complete document including Reference Table(s) and the Sequence Listing(s) can be downloaded from the EPO website

# (54) USE OF ANTI-HER3 ANTIBODY-DRUG CONJUGATE IN TREATING TUMORS AND METHOD FOR TREATING TUMORS USING ANTI-HER3 ANTIBODY-DRUG CONJUGATE

(57) The present invention relates to a use of an anti-HER3 antibody-drug conjugate in treating tumors and a method for treating tumors using the anti-HER3 antibody-drug conjugate. Specifically, the present invention relates to the use of an anti-HER3 antibody-drug conjugate in the preparation of a drug for treating EGFR-mutant tumors. The anti-HER3 antibody-drug conjugate has a structure as shown in formula I.

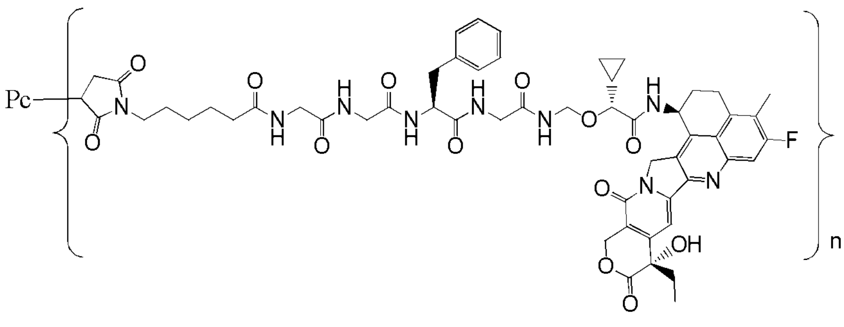

I

EP 4 782 013 A1

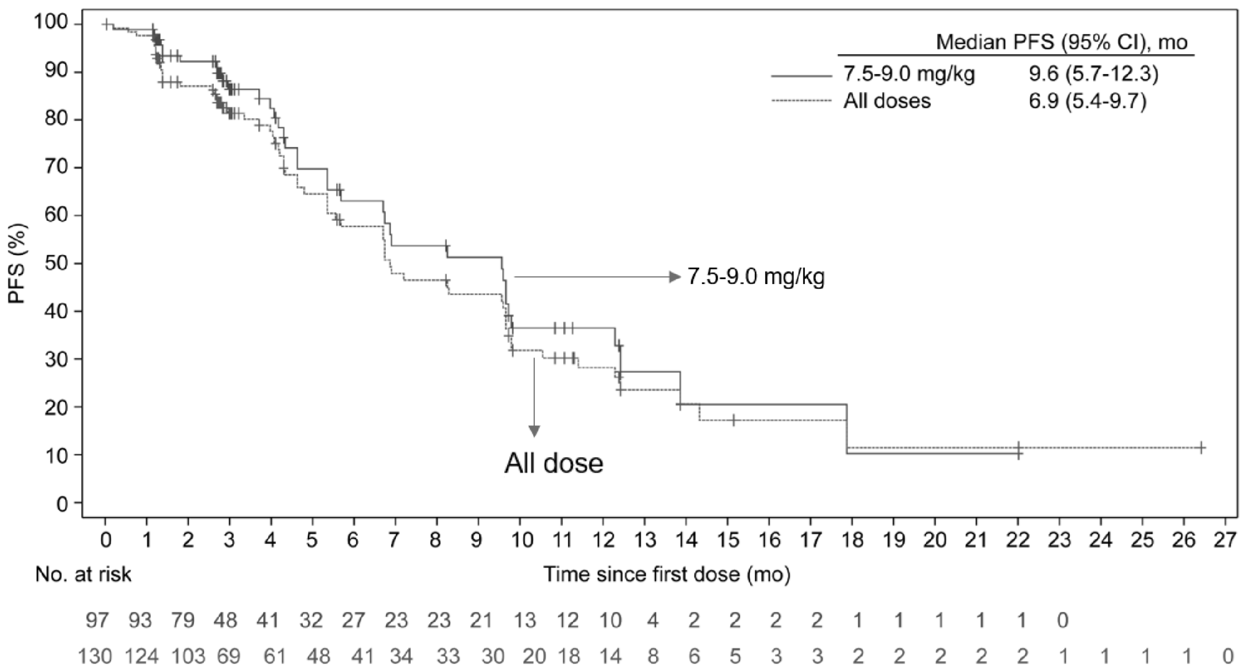
Median PFS (95% CI), mo
7.5-9.0 mg/kg 9.6 (5.7-12.3)
All doses 6.9 (5.4-9.7)
7.5-9.0 mg/kg
All dose
PFS (%)
0 10 20 30 40 50 60 70 80 90 100
0 1 2 3 4 5 6 7 8 9 10 11 12 13 14 15 16 17 18 19 20 21 22 23 24 25 26 27
Time since first dose (mo)
No. at risk
97 93 79 48 41 32 27 23 23 21 13 12 10 4 2 2 2 2 1 1 1 1 1 0
130 124 103 69 61 48 41 34 33 30 20 18 14 8 6 5 3 3 2 2 2 2 2 1 1 1 1 0

*FIG. 1*

## Description

The present invention claims priority to the Chinese patent applications with Application No. CN 2023112308116 filed on September 22, 2023 and Application No. CN 2024106793284 filed on May 29, 2024, which are incorporated by reference into the present invention.

## Technical Field

The present invention belongs to the field of medicine, and relates to a method and the medical use of an anti-HER3 antibody-drug conjugate for treating a tumor.

## Background Art

Epidermal growth factor receptor 3 (ErbB-3 or HER3) is a member of the epidermal growth factor receptor (EGFR) family. This family includes HER1 (erbB1, EGFR), HER2 (erbB2, NEU), HER3 (erbB3), and HER4 (erbB4). Each of these receptors comprises three parts, namely an extracellular region, a transmembrane region, and an intracellular region, wherein the extracellular region contains four domains, and the intracellular region contains one intracellular tyrosine kinase domain responsible for signaling and one cytoplasmic tail with tyrosine-phosphorylated residues. When a ligand binds to extracellular domains I and III, cell signaling is initiated. Under normal conditions, these receptors mediate cell division, migration, survival, and organ development. When a member of the EGFR family is mutated, the resulting aberrant signaling stimulates cell survival and is associated with cancer progression. The basic mechanism by which the HER3 receptor is activated and exerts its physiological effects is similar to that of other family members, except that its ligands include neuregulin 1 (NRG-1) and neuregulin 2 (NRG-2); and HER3 cannot form homodimers after activation, but can only form heterodimers with EGFR or HER2. During the process of forming heterodimers, the intracellular domain portion of HER3 exhibits a high level of tyrosine phosphorylation. Structural analysis has revealed that the HER3 intracellular domain contains six P85 (subunit of PI-3K) binding sites. This specific structural feature enables HER3 to recruit up to six PI-3K molecules to the regulatory subunit binding sites when interacting with P85 regulatory subunits, thereby potently activating the PI-3K signaling pathway. In fact, the HER3/HER2 dimer is the most active among the HER dimers. EGFR is widely distributed on the surface of mammalian epithelial cells, fibroblasts, glial cells, keratinocytes and other cells. The EGFR signaling pathway plays an important role in physiological processes such as cell growth, proliferation, and differentiation.

An antibody-drug conjugate (ADC) refers to a conjugate in which a cytotoxic small-molecule drug is covalently conjugated to an antibody via a chemical linker. With the antibody serving as a carrier, the small-molecule drug may be delivered to target cells in a targeted manner. ADC combines the high targeting specificity of antibodies with the potent killing of target cells by cytotoxic drugs, thus enhancing the efficacy of chemotherapy while reducing systemic exposure and associated toxicity. The endocytic efficiency of the HER3 receptor is significantly higher than that of other members of the family, making it more suitable to serve as a target for ADC drugs.

Developing safe and effective clinical therapeutic strategies for the HER3 target is an important issue to be addressed in this field.

## Summary of the Invention

The present invention provides a method and the medical use of an anti-HER3 antibody-drug conjugate for treating a tumor.

In some embodiments, the present invention provides any of the following methods or uses:

(1) use of an anti-HER3 antibody-drug conjugate in the manufacture of a medicament for treating a tumor;
(2) a method for treating a subject with a tumor, comprising administering to the subject an anti-HER3 antibody-drug conjugate; and
(3) use of an anti-HER3 antibody-drug conjugate for treating a subject with a tumor.

In some embodiments, the present invention provides any of the following methods or uses:

(1) use of an anti-HER3 antibody-drug conjugate in combination with a tyrosine kinase inhibitor in the manufacture of a medicament for treating a tumor;
(2) use of a combination of an anti-HER3 antibody-drug conjugate and a tyrosine kinase inhibitor in the manufacture of a medicament for treating a tumor;
(3) use of a pharmaceutical composition in the manufacture of a medicament for treating a tumor, wherein the

pharmaceutical composition comprises an anti-HER3 antibody-drug conjugate and a tyrosine kinase inhibitor;

(4) use of a pharmaceutical package or an article of manufacture in the manufacture of a medicament for treating a tumor, wherein the pharmaceutical package or article of manufacture comprises an anti-HER3 antibody-drug conjugate and a tyrosine kinase inhibitor;

(5) a method for treating a subject with a tumor, comprising administering to the subject an anti-HER3 antibody-drug conjugate and a tyrosine kinase inhibitor;

(6) use of an anti-HER3 antibody-drug conjugate for treating a subject with a tumor, wherein the subject is also treated with a tyrosine kinase inhibitor;

(7) use of a tyrosine kinase inhibitor for treating a subject with a tumor, wherein the subject is also treated with an anti-HER3 antibody-drug conjugate;

(8) use of an anti-HER3 antibody-drug conjugate in combination with a tyrosine kinase inhibitor for treating a tumor;

(9) use of a pharmaceutical composition for treating a tumor, wherein the pharmaceutical composition comprises an anti-HER3 antibody-drug conjugate and a tyrosine kinase inhibitor; and

(10) use of a pharmaceutical package or an article of manufacture for treating a tumor, wherein the pharmaceutical package or article of manufacture comprises an anti-HER3 antibody-drug conjugate and a tyrosine kinase inhibitor.

In some embodiments, the present invention provides a pharmaceutical composition comprising an anti-HER3 antibody-drug conjugate and a tyrosine kinase inhibitor. In some embodiments, the present invention provides a pharmaceutical package or an article of manufacture comprising an anti-HER3 antibody-drug conjugate and a tyrosine kinase inhibitor. In some embodiments, the pharmaceutical package or article of manufacture further comprises one or more containers, each of which independently comprises the anti-HER3 antibody-drug conjugate and the tyrosine kinase inhibitor.

In some embodiments, in the method or use of any of the foregoing, the tumor is selected from advanced and/or metastatic solid tumors. In some embodiments, the tumor is a locally advanced or metastatic solid tumor. In some embodiments, the "locally advanced or metastatic solid tumor" is an unresectable locally advanced or metastatic solid tumor. In some embodiments, the tumor is an unresectable locally advanced or metastatic solid tumor that fails with standard treatment or does not have an effective standard treatment regimen. In some embodiments, the tumor is non-small cell lung cancer (NSCLC).

In some specific embodiments, in the method or use of any of the foregoing, the tumor is a histologically or cytologically confirmed unresectable locally advanced or metastatic solid tumor, which has experienced recurrence or progression following standard treatment, or has no standard treatment regimen available, or is not applicable to standard treatment at this stage.

In some embodiments, in the method or use of any of the foregoing, the tumor has brain metastasis, bone metastasis, and/or liver metastasis.

In some embodiments, in the method or use of any of the foregoing, the tumor is an EGFR-mutant tumor. In some embodiments, the tumor is EGFR-mutant non-small cell lung cancer.

In some embodiments, in the method or use of any of the foregoing, the tumor is an EGFR-mutant locally advanced or metastatic tumor. In some embodiments, the tumor is EGFR-mutant locally advanced or metastatic non-small cell lung cancer.

In some embodiments, the "EGFR mutation" and "EGFR mutation positive" may be used interchangeably, which may be a sensitive mutation or a non-sensitive mutation. In some embodiments, the "EGFR mutation", as confirmed with histological or blood specimens, comprises at least one of the following EGFR mutations: exon 19 deletion (Ex19del), exon 21 substitution mutation (L858R mutation), G719X, and L861Q.

In some embodiments, in the method or use of any of the foregoing, the tumor is a tumor that has experienced treatment failure with a tyrosine kinase inhibitor (TKI). In some embodiments, the tumor is non-small cell lung cancer that has experienced treatment failure with a tyrosine kinase inhibitor (TKI).

In some embodiments, in the method or use of any of the foregoing, the tumor is an EGFR-mutant tumor that has experienced treatment failure with a tyrosine kinase inhibitor (TKI). In some embodiments, the tumor is EGFR-mutant non-small cell lung cancer that has experienced treatment failure with a tyrosine kinase inhibitor (TKI).

In some embodiments, in the method or use of any of the foregoing, the tumor is a locally advanced or metastatic tumor that has experienced treatment failure with a tyrosine kinase inhibitor (TKI). In some embodiments, the tumor is locally advanced or metastatic non-small cell lung cancer that has experienced treatment failure with a tyrosine kinase inhibitor (TKI).

In some embodiments, in the method or use of any of the foregoing, the tumor is an EGFR-mutant locally advanced or metastatic tumor that has experienced treatment failure with a tyrosine kinase inhibitor (TKI). In some embodiments, the tumor is EGFR-mutant locally advanced or metastatic non-small cell lung cancer that has experienced treatment failure with a tyrosine kinase inhibitor (TKI).

In some embodiments, locally advanced or metastatic is defined as being staged as Stage IIIB to Stage IV in

accordance with the criteria of the 9th edition of the TNM Staging System of the International Association for the Study of Lung Cancer (IASLC), and no longer amenable to radical surgery or radical chemoradiotherapy.

In the present invention, "treatment failure with a TKI", "treatment failure with an EGFR-TKI", and "resistance to an EGFR-TKI" may be used interchangeably. In some embodiments, "treatment failure" refers to the occurrence of radiological disease progression during or after treatment.

In some embodiments, the tumor includes, but is not limited to, any one or any combination of the following:

(1) locally advanced or metastatic solid tumors (e.g., locally advanced or metastatic NSCLC);

(2) EGFR mutation-positive non-small cell lung cancer (e.g., EGFR mutation-positive locally advanced or metastatic non-small cell lung cancer, or naïve EGFR mutation-positive locally advanced or metastatic non-small cell lung cancer);

(3) non-small cell lung cancer that has experienced treatment failure with an EGFR-TKI (e.g., locally advanced or metastatic non-small cell lung cancer that has experienced treatment failure with an EGFR-TKI, or EGFR mutation-positive locally advanced or metastatic non-small cell lung cancer that has experienced treatment failure with an EGFR-TKI);

(4) advanced or metastatic solid tumors that have experienced recurrence or progression following standard treatment, or have no standard treatment regimen available, or are not applicable to standard treatment at this stage; and

(5) unresectable locally advanced or metastatic solid tumors that have experienced recurrence or progression following standard treatment, or have no standard treatment regimen available, or are not applicable to standard treatment at this stage.

In some embodiments, the subject has received treatment with an EGFR TKI for locally advanced or metastatic NSCLC. In some embodiments, a subject with the T790M mutation must have received treatment with a third-generation EGFR TKI, and has experienced radiological disease progression during or after the treatment. By way of example, the subject includes, but is not limited to:

(1) subjects who have experienced tumor recurrence or metastasis during adjuvant therapy with an EGFR TKI or within 6 months of discontinuation of such therapy; (2) subjects with non-metastatic tumors, who have previously received neoadjuvant/adjuvant chemotherapy or radical chemoradiotherapy, experienced tumor recurrence or metastasis at least 6 months after completion of the aforementioned treatment, received treatment with an EGFR TKI, and experienced radiological disease progression during or after the treatment; and (3) subjects with locally advanced or metastatic NSCLC, who are permitted to receive anti-angiogenic therapy or targeted therapy during treatment with an EGFR TKI (e.g., EGFR/c-MET bispecific antibodies, excluding antibody-drug conjugates), while administration of other systemic anti-tumor therapies other than the aforementioned treatments is not permitted.

In some embodiments, "locally advanced or metastatic NSCLC" refers to unresectable locally advanced or metastatic non-small cell lung cancer that is histologically or cytologically confirmed. In some specific embodiments, "locally advanced or metastatic NSCLC" refers to unresectable locally advanced or metastatic non-small cell lung cancer that is histologically or cytologically confirmed, which has experienced recurrence or progression following standard treatment, or has no standard treatment regimen available, or is not applicable to standard treatment at this stage.

In some embodiments, "EGFR mutation-positive locally advanced or metastatic non-small cell lung cancer" refers to unresectable locally advanced or metastatic non-small cell lung cancer that is histologically or cytologically confirmed, and confirmed to have an EGFR mutation (sensitive or non-sensitive mutation) with histological or blood specimens. In some embodiments, "EGFR mutation-positive locally advanced or metastatic non-small cell lung cancer" refers to unresectable locally advanced or metastatic non-small cell lung cancer that is histologically or cytologically confirmed, and confirmed to have EGFR exon 19 deletion or L858R mutation or G719X or L861Q with histological or blood specimens. In some embodiments, "EGFR mutation-positive locally advanced or metastatic non-small cell lung cancer" is "EGFR mutation-positive locally advanced or metastatic non-small cell lung cancer that has experienced treatment failure with an EGFR-TKI" or "naïve EGFR mutation-positive locally advanced or metastatic non-small cell lung cancer".

In the present invention, standard treatment regimens are well known to those skilled in the art, such as the treatment regimens disclosed in treatment guidelines approved by regulatory authorities. Examples include, but are not limited to, the detailed and up-to-date information on standard treatment regimens for various cancers provided in the NCCN Guidelines and CSCO Guidelines.

Anti-HER3 Antibody-Drug Conjugate

In some embodiments, the anti-HER3 antibody-drug conjugate is administered at a dose of about 0.1 mg/kg to about 20 mg/kg, such as about 1.0 mg/kg to about 20 mg/kg, about 1.0 mg/kg to about 12 mg/kg, about 1.5 mg/kg to about 12 mg/kg, about 1.0 mg/kg to about 10.5 mg/kg, about 1.0 mg/kg to about 10 mg/kg, about 1.0 mg/kg to about 9 mg/kg,

about 1.5 mg/kg to about 10.5 mg/kg, about 1.5 mg/kg to about 9.0 mg/kg, about 3.0 mg/kg to about 9.0 mg/kg, about 4.5 mg/kg to about 9.0 mg/kg, about 6.0 mg/kg to about 9.0 mg/kg, about 7.5 mg/kg to about 9.0 mg/kg, about 1.5 mg/kg to about 7.5 mg/kg, about 3.0 mg/kg to about 7.5 mg/kg, or any range between these point values. In some embodiments, the anti-HER3 antibody-drug conjugate is administered at a dose selected from about 1.0 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.6 mg/kg, about 2.7 mg/kg, about 2.8 mg/kg, about 2.9 mg/kg, about 3.0 mg/kg, about 3.1 mg/kg, about 3.2 mg/kg, about 3.4 mg/kg, about 3.5 mg/kg, about 3.6 mg/kg, about 3.8 mg/kg, about 4.0 mg/kg, about 4.2 mg/kg, about 4.3 mg/kg, about 4.5 mg/kg, about 4.6 mg/kg, about 4.8 mg/kg, about 5.0 mg/kg, about 5.1 mg/kg, about 5.3 mg/kg, about 5.5 mg/kg, about 5.6 mg/kg, about 5.8 mg/kg, about 6.0 mg/kg, about 6.1 mg/kg, about 6.3 mg/kg, about 6.4 mg/kg, about 6.5 mg/kg, about 6.6 mg/kg, about 6.8 mg/kg, about 6.9 mg/kg, about 7.0 mg/kg, about 7.2 mg/kg, about 7.4 mg/kg, about 7.5 mg/kg, about 7.6 mg/kg, about 7.7 mg/kg, about 7.8 mg/kg, about 7.9 mg/kg, about 8.0 mg/kg, about 8.1 mg/kg, about 8.2 mg/kg, about 8.3 mg/kg, about 8.4 mg/kg, about 8.5 mg/kg, about 8.6 mg/kg, about 8.7 mg/kg, about 8.8 mg/kg, about 8.9 mg/kg, about 9.0 mg/kg, about 9.1 mg/kg, about 9.2 mg/kg, about 9.3 mg/kg, about 9.4 mg/kg, about 9.5 mg/kg, about 10.0 mg/kg, about 10.5 mg/kg, about 12 mg/kg, about 12.5 mg/kg, about 13 mg/kg, about 13.5 mg/kg, about 14 mg/kg, about 16 mg/kg, about 18 mg/kg, about 19 mg/kg, or about 20 mg/kg.

In some embodiments, the anti-HER3 antibody-drug conjugate is administered at a frequency of once a week, once every two weeks, once every three weeks, once every four weeks, once every six weeks, once every eight weeks, once every ten weeks, or once every twelve weeks. In some specific embodiments, the anti-HER3 antibody-drug conjugate is administered at a frequency of once every three weeks.

In some embodiments, the administration regimen of the anti-HER3 antibody-drug conjugate is selected from any one of the following:

($a_1$) the administration dose is about 0.1 mg/kg to about 20 mg/kg, and the administration frequency is once a week, once every two weeks, once every three weeks, once every four weeks, once every six weeks, once every eight weeks, once every ten weeks, or once every twelve weeks;

($a_2$) the administration dose is about 1.0 mg/kg to about 13.5 mg/kg, and the administration frequency is selected from once a week, once every two weeks, once every three weeks, once every four weeks, once every six weeks, once every eight weeks, or once every ten weeks;

($a_3$) the administration dose is about 1.0 mg/kg to about 10.5 mg/kg, and the administration frequency is selected from once a week, once every two weeks, once every three weeks, once every four weeks, once every six weeks, once every eight weeks, or once every ten weeks;

($a_4$) the administration dose is about 1.0 mg/kg to about 9.0 mg/kg, and the administration frequency is selected from once a week, once every two weeks, once every three weeks, once every four weeks, once every six weeks, once every eight weeks, or once every ten weeks;

($a_5$) the administration dose is about 1.5 mg/kg to about 10.5 mg/kg, and the administration frequency is selected from once a week, once every two weeks, once every three weeks, once every four weeks, once every six weeks, once every eight weeks, or once every ten weeks;

($a_6$) the administration dose is about 1.5 mg/kg to about 9.0 mg/kg, and the administration frequency is selected from once a week, once every two weeks, once every three weeks, once every four weeks, once every six weeks, once every eight weeks, or once every ten weeks;

($a_7$) the administration dose is about 1.5 mg/kg to about 12.0 mg/kg, and the administration frequency is selected from once a week, once every two weeks, once every three weeks, once every four weeks, once every six weeks, once every eight weeks, or once every ten weeks;

($a_8$) the administration dose is about 7.5 mg/kg to about 9.0 mg/kg, and the administration frequency is selected from once a week, once every two weeks, once every three weeks, once every four weeks, once every six weeks, once every eight weeks, or once every ten weeks;

($a_9$) the administration dose is about 1.0 mg/kg, about 1.5 mg/kg, about 3.0 mg/kg, about 4.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 10.5 mg/kg, about 12 mg/kg, or about 13.5 mg/kg, and the administration frequency is selected from once a week, once every two weeks, once every three weeks, once every four weeks, once every six weeks, once every eight weeks, or once every ten weeks;

($a_{10}$) the administration dose is about 1.5 mg/kg, about 3.0 mg/kg, about 4.5 mg/kg, about 6.0 mg/kg, about 6.5 mg/kg, about 7.0 mg/kg, about 7.5 mg/kg, about 8.0 mg/kg, about 8.5 mg/kg, or about 9.0 mg/kg, and the administration frequency is selected from once a week, once every two weeks, once every three weeks, once every four weeks, once every six weeks, once every eight weeks, or once every ten weeks;

($a_{11}$) the anti-HER3 antibody-drug conjugate is administered at a dose of about 1.5 mg/kg, and at a frequency of once every three weeks;

($a_{12}$) the anti-HER3 antibody-drug conjugate is administered at a dose of about 3.0 mg/kg, and at a frequency of once every three weeks;

($a_{13}$) the anti-HER3 antibody-drug conjugate is administered at a dose of about 4.5 mg/kg, and at a frequency of once every three weeks;

($a_{14}$) the anti-HER3 antibody-drug conjugate is administered at a dose of about 6.0 mg/kg, and at a frequency of once every three weeks;

($a_l$s) the anti-HER3 antibody-drug conjugate is administered at a dose of about 7.5 mg/kg, and at a frequency of once every three weeks;

($a_{16}$) the anti-HER3 antibody-drug conjugate is administered at a dose of about 8.0 mg/kg, and at a frequency of once every three weeks;

($a_{17}$) the anti-HER3 antibody-drug conjugate is administered at a dose of about 8.5 mg/kg, and at a frequency of once every three weeks; and

($a_{18}$) the anti-HER3 antibody-drug conjugate is administered at a dose of about 9.0 mg/kg, and at a frequency of once every three weeks.

In some specific embodiments, in the administration regimen of any one of ($a_1$) to ($a_{10}$) above, the anti-HER3 antibody-drug conjugate is administered at a frequency of once every three weeks.

In some embodiments, the anti-HER3 antibody-drug conjugate is derived from an antibody-drug conjugate of any of the structures in WO 2022078425 A1 or WO 2020063676 A1, and the structures, preparation methods and other relevant contents of the immunoconjugates in the aforementioned patents are incorporated by reference into the present invention.

In some embodiments, the anti-HER3 antibody-drug conjugate has a structure as shown in the formula below:

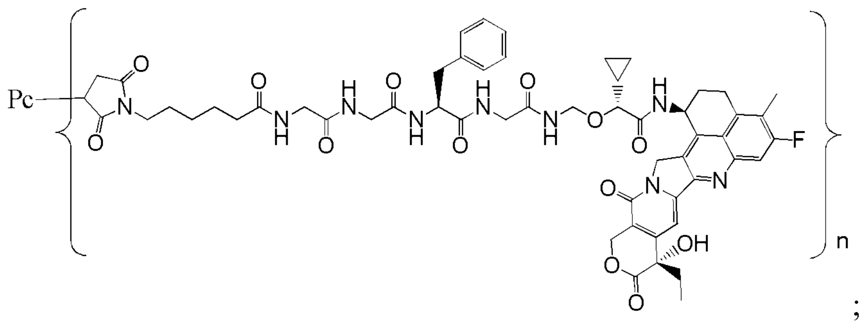

;

wherein: n is 1 to 8, and n is a decimal or an integer; and Pc is an anti-HER3 antibody.

In some embodiments, n is 4.0 ± 0.4.

In some embodiments, the anti-HER3 antibody-drug conjugate has a structure as shown in the formula below:

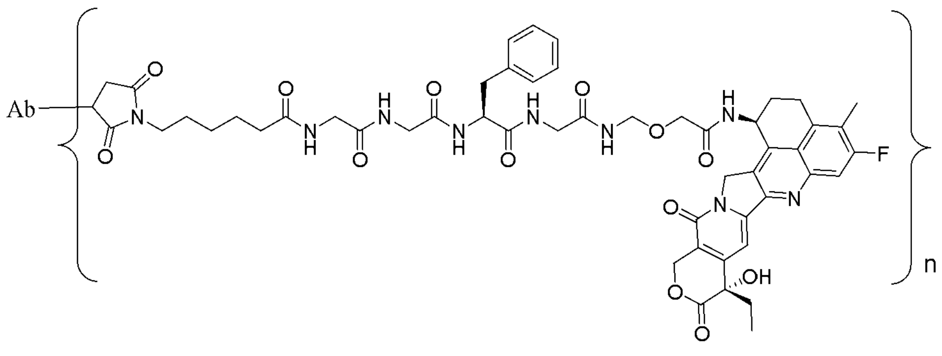

;

wherein: n is 1 to 8, and n is a decimal or an integer; and Pc is an anti-HER3 antibody.

In some embodiments, n is 4.0 ± 0.4.

In the present invention, "antibody" is used in the broadest sense, which encompasses various antibody structures, including but not limited to monoclonal antibodies, polyclonal antibodies, multispecific antibodies (e.g., bispecific antibodies), full-length antibodies, or antigen-binding fragments thereof (also known as "antigen-binding portions"), as long as they exhibit the desired antigen-binding activity.

In some embodiments, the anti-HER3 antibody is derived from any type of anti-HER3 antibody or antigen-binding

fragments thereof in WO 2022078425 A1, and the antibody sequences, preparation methods and other relevant contents in the aforementioned patent are incorporated by reference into the present invention.

In some embodiments, the anti-HER3 antibody comprises a heavy chain variable region (VH) and a light chain variable region (VL), wherein the heavy chain variable region comprises HCDR1, HCDR2, and HCDR3 as set forth in SEQ ID NO: 1, SEQ ID NO: 2, and SEQ ID NO: 3, respectively; and/or the light chain variable region comprises LCDR1, LCDR2, and LCDR3 as set forth in SEQ ID NO: 4, SEQ ID NO: 5, and SEQ ID NO: 6, respectively.

Each of the foregoing CDR sequences is as shown in the table below.

Table 1. CDR sequences obtained by the Kabat numbering scheme

| Antibody | HER3-29 | SEQ ID NO. |
|---|---|---|
| Heavy chain CDR1 | DYAMH | SEQ ID NO: 1 |
| Heavy chain CDR2 | GISWNSGSIGYADSVKG | SEQ ID NO: 2 |
| Heavy chain CDR3 | EGLPGLDY | SEQ ID NO: 3 |
| Light chain CDR1 | RASQHVGTYLN | SEQ ID NO: 4 |
| Light chain CDR2 | GAANLQS | SEQ ID NO: 5 |
| Light chain CDR3 | QQSYNTPPFS | SEQ ID NO: 6 |

The CDRs are defined according to the Kabat numbering system.

In some embodiments, the anti-HER3 antibody comprises any one, any two, any three, any four, any five, or all six CDRs selected from HCDR1, HCDR2, HCDR3, LCDR1, LCDR2, and LCDR3 as set forth in SEQ ID NO: 1 to SEQ ID NO: 6.

In some embodiments, the anti-HER3 antibody comprises a heavy chain variable region (VH) and a light chain variable region (VL), wherein the heavy chain variable region comprises HCDR1, HCDR2, and HCDR3 as set forth in SEQ ID NO: 7, and the light chain variable region comprises LCDR1, LCDR2, and LCDR3 as set forth in SEQ ID NO: 8. The CDRs are defined according to the Kabat, IMGT, Chothia, AbM, or Contact numbering system. In some specific embodiments, the CDRs are defined according to the Kabat numbering system.

In some embodiments, the anti-HER3 antibody is a murine antibody, a chimeric antibody, a humanized antibody, a human antibody, or an antigen-binding fragment of any of the foregoing. In some specific embodiments, the anti-HER3 antibody is selected from a humanized antibody or an antigen-binding fragment thereof.

In some embodiments, in the anti-HER3 antibody, the VH comprises an amino acid sequence as set forth in SEQ ID NO: 7 or an amino acid sequence having at least 80% or 90% sequence identity thereto; and the VL comprises an amino acid sequence as set forth in SEQ ID NO: 8 or an amino acid sequence having at least 80% or 90% sequence identity thereto.

HER3 heavy chain variable region:

QVQLVQSGGGLVQPGRSLRLSCAASGFTFDDYAMHWVRQAPGKGLEWV
SGISWNSGSIGYADSVKGRFTISRDNAKNSLYLQMNSLRAEDTALYYCAK
EGLPGLDYWGQGTLVTVSS

SEQ ID NO: 7

HER3 light chain variable region:

DIQMTQSPSSLSASIGDRATITCRASQHVGTYLNWYQQKPGKTPKLLISGA
ANLQSGVPSRFSGSGSGTDFTLTISSLQPEDFATYYCQQSYNTPPFSFGQGT
KVEIK

SEQ ID NO: 8

In some embodiments, the anti-HER3 antibody comprises any one or two of the VH and VL above.

In some embodiments, the anti-HER3 antibody further comprises an antibody constant region. For example, the

heavy chain constant region portion of the antibody constant region is selected from human IgG1, IgG2, IgG3, and IgG4 constant regions and variants thereof; and the light chain constant region portion of the antibody constant region is selected from the human antibody kappa and lambda chain constant regions and variants thereof.

The aforementioned light chain variable region is combined with the light chain constant region sequence to form a light chain sequence, and the heavy chain variable region is combined with the heavy chain constant region to form a heavy chain sequence. Exemplary humanized antibody sequences are as shown below.

HER3-29 heavy chain:

QVQLVQSGGGLVQPGRSLRLSCAASGFTFDDYAMHWVRQAPGKGLEWV
SGISWNSGSIGYADSVKGRFTISRDNAKNSLYLQMNSLRAEDTALYYCAK
EGLPGLDYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK

SEQ ID NO: 9

HER3-29 light chain:

DIQMTQSPSSLSASIGDRATITCRASQHVGTYLNWYQQKPGKTPKLLISGA
ANLQSGVPSRFSGSGSGTDFTLTISSLQPEDFATYYCQQSYNTPPFSFGQGT
KVEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSP
VTKSFNRGEC

SEQ ID NO: 10

In some embodiments, the anti-HER3 antibody comprises a heavy chain and a light chain, wherein the heavy chain comprises an amino acid sequence as set forth in SEQ ID NO: 9 or an amino acid sequence having at least 80% or 90% identity thereto, and the light chain comprises an amino acid sequence as set forth in SEQ ID NO: 10 or an amino acid sequence having at least 80% or 90% identity thereto.

In some embodiments, the anti-HER3 antibody comprises any one or two of the foregoing heavy chain and light chain.

In the context of the present invention, "at least 80%" and "at least 90%" encompass 80% and above, such as at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and a numerical range between any two of the foregoing.

In some embodiments, the administration route of the anti-HER3 antibody-drug conjugate is oral administration, parenteral administration, or transdermal administration. Such parenteral administration includes, but is not limited to, intravenous injection, subcutaneous injection, and intramuscular injection. In some specific embodiments, the administration route of the anti-HER3 antibody-drug conjugate is intravenous injection administration.

In some embodiments, the anti-HER3 antibody-drug conjugate is formulated as an injectable form. By way of example, the injectable form of the anti-HER3 antibody-drug conjugate is an injectable solution or a lyophilized powder for injection, which comprises the anti-HER3 antibody-drug conjugate and one or more pharmaceutically acceptable excipients.

In some embodiments, the pharmaceutical composition comprising the anti-HER3 antibody-drug conjugate further comprises a buffer, a stabilizer, an osmotic pressure regulator, and/or a surfactant. By way of example, the buffer is selected from any one of acetic acid-sodium acetate, succinic acid-sodium succinate, histidine hydrochloride, and citric acid-sodium citrate buffers. In some specific embodiments, the buffer is acetic acid-sodium acetate. By way of example, the surfactant may be selected from polysorbate (e.g., polysorbate 20 and polysorbate 80), poloxamer, Triton, sodium dodecyl sulfonate, sodium lauryl sulfonate, sodium octyl glucoside, lauryl sulfobetaine, myristyl sulfobetaine, linoleyl sulfobetaine, stearyl sulfobetaine, lauryl sarcosine, myristyl sarcosine, linoleyl sarcosine, stearyl sarcosine, linoleyl betaine, myristyl betaine, cetyl betaine, lauramidopropyl betaine, cocamidopropyl betaine, linoleamidopropyl betaine, myristamidopropyl betaine, palmitamidopropyl betaine, isostearamidopropyl betaine, myristamidopropyl dimethylamine, palmitamidopropyl dimethylamine, isostearamidopropyl dimethylamine, sodium methyl cocoyl, sodium methyl oleoyl taurate, polyethylene glycol, polypropylene glycol, copolymers of ethylene glycol and propylene glycol, and the like. In some specific embodiments, the surfactant is polysorbate. Examples are polysorbate 80 or polysorbate 20. In some specific embodiments, the surfactant is polysorbate 80. In some embodiments, the pharmaceutical composition comprising the anti-HER3 antibody-drug conjugate further comprises a sugar. By way of example, the "sugar" includes a conventional compound of formula $(CH_2O)_n$ and derivatives thereof, including monosaccharides, disaccharides, trisaccharides, polysaccharides, sugar alcohols, reducing sugars, non-reducing sugars, and the like. The sugar may be selected from glucose, sucrose, trehalose, alpha,alpha-trehalose dihydrate, lactose, fructose, maltose, dextran, glycerol, erythritol, glycerin, arabitol, sylitol, sorbitol, mannitol, melibiose, melezitose, raffinose, manninotriose, stachyose, maltose, lactulose, maltulose, sorbitol, maltitol, lactitol, isomaltulose, and the like. In some specific embodiments, the "sugar" is sucrose.

Anti-HER3 Antibody-Drug Conjugate in Combination with Tyrosine Kinase Inhibitor

In some embodiments, the present invention provides use of an anti-HER3 antibody-drug conjugate in combination with a tyrosine kinase inhibitor in the manufacture of a medicament for treating a tumor.

In some embodiments, the anti-HER3 antibody-drug conjugate is used in combination with the tyrosine kinase inhibitor to treat the tumor.

In some embodiments, the present invention provides a method for treating a subject with a tumor, comprising administering to the subject 1) an anti-HER3 antibody-drug conjugate and 2) a tyrosine kinase inhibitor.

In some embodiments, the tyrosine kinase inhibitor is selected from neratinib, lapatinib, tucatinib, gefitinib, erlotinib, icotinib, afatinib, dacomitinib, osimertinib, or almonertinib.

In some embodiments, the tyrosine kinase inhibitor is almonertinib.

In some embodiments, the tyrosine kinase inhibitor is administered at a dose of about 1 mg to about 1000 mg, such as about 10 mg to about 1000 mg, about 10 mg to about 900 mg, about 10 mg to about 800 mg, about 10 mg to about 500 mg, about 50 mg to about 500 mg, about 50 mg to about 450 mg, about 50 mg to about 400 mg, about 50 mg to about 350 mg, about 50 mg to about 300 mg, about 50 mg to about 250 mg, about 50 mg to about 200 mg, about 50 mg to about 180 mg, about 50 mg to about 150 mg, about 55 mg to about 110 mg, about 100 mg to about 150 mg, about 100 mg to about 180 mg, or about 100 mg to about 200 mg, and a numerical range between any two of the foregoing.

In some embodiments, the tyrosine kinase inhibitor is administered at a dose of about 50 mg, about 55 mg, about 60 mg, about 70 mg, about 80 mg, about 90 mg, about 95 mg, about 100 mg, about 110 mg, about 120 mg, about 130 mg, about 140 mg, about 150 mg, about 160 mg, about 170 mg, about 180 mg, about 190 mg, about 200 mg, about 220 mg, about 240 mg, about 260 mg, about 280 mg, about 300 mg, about 350 mg, about 400 mg, about 500 mg, about 600 mg, about 700 mg, about 800 mg, about 900 mg, or about 1000 mg.

In some embodiments, the tyrosine kinase inhibitor is administered at a frequency of once a day, twice a day, three times a day, once every two days, once every three days, once every four days, or once every five days. In some specific embodiments, the tyrosine kinase inhibitor is administered at a frequency of once a week.

In some embodiments, the administration regimen of the tyrosine kinase inhibitor is selected from any one of the following:

($b_1$) the tyrosine kinase inhibitor is administered at a dose of about 10 mg to about 1000 mg, and at a frequency of once a day, twice a day, three times a day, once every two days, once every three days, once every four days, once every five days, or once a week;

($b_2$) the tyrosine kinase inhibitor is administered at a dose of about 10 mg to about 500 mg, and at a frequency of once a day, twice a day, three times a day, once every two days, once every three days, once every four days, once every five days, or once a week;

($b_3$) the tyrosine kinase inhibitor is administered at a dose of about 50 mg to about 200 mg, and at a frequency of once a day, twice a day, three times a day, once every two days, once every three days, once every four days, once every five days, or once a week;

($b_4$) the tyrosine kinase inhibitor is administered at a dose of about 50 mg to about 150 mg, and at a frequency of once a day, twice a day, three times a day, once every two days, once every three days, once every four days, once every five days, or once a week;

($b_5$) the tyrosine kinase inhibitor is administered at a dose of about 55 mg to about 110 mg, and at a frequency of once a day, twice a day, three times a day, once every two days, once every three days, once every four days, once every five days, or once a week;

($b_6$) the tyrosine kinase inhibitor is administered at a dose of about 100 mg to about 200 mg, and at a frequency of once a day, twice a day, three times a day, once every two days, once every three days, once every four days, once every five days, or once a week;

($b_7$) the tyrosine kinase inhibitor is administered at a dose of about 55 mg, and at a frequency of once a day, twice a day, three times a day, once every two days, once every three days, once every four days, once every five days, or once a week; and

($b_8$) the tyrosine kinase inhibitor is administered at a dose of about 110 mg, and at a frequency of once a day, twice a day, three times a day, once every two days, once every three days, once every four days, once every five days, or once a week.

In some embodiments, in the administration regimen of any one of ($b_1$) to ($b_8$) above, the tyrosine kinase inhibitor is administered at a frequency of once a day.

In some embodiments, the administration frequency and dose of the anti-HER3 antibody-drug conjugate are as shown in any one of the above.

By way of example, the anti-HER3 antibody-drug conjugate is administered at a dose of about 0.1 mg/kg to about 20 mg/kg, such as about 1.0 mg/kg to about 20 mg/kg, about 1.0 mg/kg to about 13.5 mg/kg, about 1.0 mg/kg to about 12 mg/kg, about 1.5 mg/kg to about 12 mg/kg, about 1.0 mg/kg to about 10.5 mg/kg, about 1.0 mg/kg to about 10 mg/kg, about 1.0 mg/kg to about 9 mg/kg, about 1.5 mg/kg to about 10.5 mg/kg, about 1.5 mg/kg to about 9.0 mg/kg, about 3.0 mg/kg to about 9.0 mg/kg, about 4.5 mg/kg to about 9.0 mg/kg, about 6.0 mg/kg to about 9.0 mg/kg, about 7.5 mg/kg to about 9.0 mg/kg, about 1.5 mg/kg to about 7.5 mg/kg, or about 3.0 mg/kg to about 7.5 mg/kg, e.g., about 1.5 mg/kg, about 3.0 mg/kg, about 4.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 9.5 mg/kg, about 10.0 mg/kg, about 10.5 mg/kg, about 12.0 mg/kg, about 13.5 mg/kg, and the like. The anti-HER3 antibody-drug conjugate is administered at a frequency of once a week, once every two weeks, once every three weeks, once every four weeks, once every six weeks, once every eight weeks, once every ten weeks, or once every twelve weeks, such as once every three weeks.

By way of example, the administration regimen of the anti-HER3 antibody-drug conjugate is selected from the administration regimen of any one of ($a_1$) to ($a_{14}$).

In some embodiments, the administration regimen of the anti-HER3 antibody-drug conjugate in combination with the tyrosine kinase inhibitor is selected from any one of the following:

($a_1$) the anti-HER3 antibody-drug conjugate is administered at a dose of about 0.1 mg/kg to about 20 mg/kg, and at a frequency of once a week, once every two weeks, once every three weeks, once every four weeks, once every six weeks, once every eight weeks, once every ten weeks, or once every twelve weeks; and

($b_1$) the tyrosine kinase inhibitor is administered at a dose of about 10 mg to about 1000 mg, and at a frequency of once a day, twice a day, three times a day, once every two days, once every three days, once every four days, once every five days, or once a week;

or

($a_2$) the anti-HER3 antibody-drug conjugate is administered at a dose of about 1.0 mg/kg to about 20 mg/kg, and at a frequency selected from once a week, once every two weeks, once every three weeks, once every four weeks, once every six weeks, once every eight weeks, or once every ten weeks; and

($b_2$) the tyrosine kinase inhibitor is administered at a dose of about 10 mg to about 500 mg, and at a frequency of once a day, twice a day, three times a day, once every two days, once every three days, once every four days, once every five days, or once a week;

or

($a_3$) the anti-HER3 antibody-drug conjugate is administered at a dose of about 1.0 mg/kg to about 13.5 mg/kg, and at a frequency selected from once a week, once every two weeks, once every three weeks, once every four weeks, once every six weeks, once every eight weeks, or once every ten weeks; and

($b_3$) the tyrosine kinase inhibitor is administered at a dose of about 50 mg to about 200 mg, and at a frequency of once a day, twice a day, three times a day, once every two days, once every three days, once every four days, once every five days, or once a week;

or

($a_4$) the anti-HER3 antibody-drug conjugate is administered at a dose of about 1.0 mg/kg to about 9.0 mg/kg, and at a

frequency selected from once a week, once every two weeks, once every three weeks, once every four weeks, once every six weeks, once every eight weeks, or once every ten weeks; and

($b_3$) the tyrosine kinase inhibitor is administered at a dose of about 50 mg to about 200 mg, and at a frequency of once a day, twice a day, three times a day, once every two days, once every three days, once every four days, once every five days, or once a week;

or

($a_5$) the anti-HER3 antibody-drug conjugate is administered at a dose of about 1.5 mg/kg to about 12.5 mg/kg, and at a frequency selected from once a week, once every two weeks, once every three weeks, once every four weeks, once every six weeks, once every eight weeks, or once every ten weeks; and

($b_3$) the tyrosine kinase inhibitor is administered at a dose of about 50 mg to about 200 mg, and at a frequency of once a day, twice a day, three times a day, once every two days, once every three days, once every four days, once every five days, or once a week;

or

($a_5$) the anti-HER3 antibody-drug conjugate is administered at a dose of about 1.5 mg/kg to about 10.5 mg/kg, and at a frequency selected from once a week, once every two weeks, once every three weeks, once every four weeks, once every six weeks, once every eight weeks, or once every ten weeks; and

($b_4$) the tyrosine kinase inhibitor is administered at a dose of about 50 mg to about 150 mg, and at a frequency of once a day, twice a day, three times a day, once every two days, once every three days, once every four days, once every five days, or once a week;

or

($a_6$) the anti-HER3 antibody-drug conjugate is administered at a dose of about 7.5 mg/kg to about 9.0 mg/kg, and at a frequency selected from once a week, once every two weeks, once every three weeks, once every four weeks, once every six weeks, once every eight weeks, or once every ten weeks; and

($b_5$) the tyrosine kinase inhibitor is administered at a dose of about 55 mg to about 110 mg, and at a frequency of once a day, twice a day, three times a day, once every two days, once every three days, once every four days, once every five days, or once a week;

or

the anti-HER3 antibody-drug conjugate is administered at a dose of about 1.5 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 3.0 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 4.5 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 6.0 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 6.5 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 7.5 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 8.0 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 8.5 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 9.0 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 10.5 mg/kg, and at a frequency of once every three weeks; and the tyrosine kinase inhibitor is administered at a dose of about 110 mg, and at a frequency of once a day;

or

the anti-HER3 antibody-drug conjugate is administered at a dose of about 1.5 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 3.0 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 4.5 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 6.0 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 6.5 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 7.5 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 8.0 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 8.5 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 9.0 mg/kg, and at a frequency of once every three weeks; and the tyrosine kinase inhibitor is administered at a dose of about 55 mg, and at a frequency of once a day;

or

the anti-HER3 antibody-drug conjugate is administered at a dose of about 10.5 mg/kg, and at a frequency of once every three weeks; and the tyrosine kinase inhibitor is administered at a dose of about 55 mg, and at a frequency of once a day.

In some embodiments, the anti-HER3 antibody-drug conjugate is an anti-HER3 antibody-drug conjugate of any of the foregoing structures.

By way of example, the anti-HER3 antibody-drug conjugate has a structure as shown in the formula below:

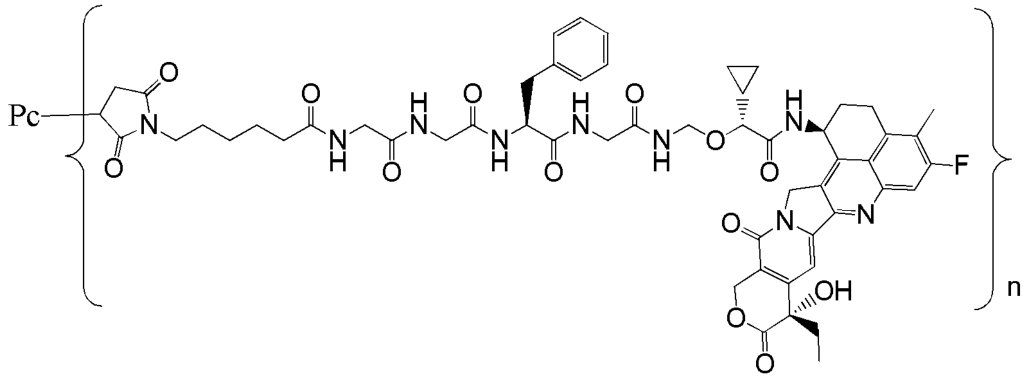

;

wherein: n is 1 to 8, and n is a decimal or an integer; and Pc is an anti-HER3 antibody.

By way of example, in some embodiments, n is 4.0 ± 0.4.

By way of example, in some embodiments, the anti-HER3 antibody comprises a heavy chain variable region (VH) and a light chain variable region (VL), wherein the heavy chain variable region comprises HCDR1, HCDR2, and HCDR3 as set forth in SEQ ID NO: 1, SEQ ID NO: 2, and SEQ ID NO: 3, respectively; and/or the light chain variable region comprises LCDR1, LCDR2, and LCDR3 as set forth in SEQ ID NO: 4, SEQ ID NO: 5, and SEQ ID NO: 6, respectively.

By way of example, in some embodiments, in the anti-HER3 antibody, the VH comprises an amino acid sequence as set forth in SEQ ID NO: 7 or an amino acid sequence having at least 80% or 90% sequence identity thereto; and the VL comprises an amino acid sequence as set forth in SEQ ID NO: 8 or an amino acid sequence having at least 80% or 90% sequence identity thereto.

By way of example, in some embodiments, the anti-HER3 antibody comprises a heavy chain and a light chain, wherein the heavy chain comprises an amino acid sequence as set forth in SEQ ID NO: 9 or an amino acid sequence having at least 80% or 90% identity thereto, and the light chain comprises an amino acid sequence as set forth in SEQ ID NO: 10 or an amino acid sequence having at least 80% or 90% identity thereto.

In some embodiments, the administration route of the tyrosine kinase inhibitor is oral administration, parenteral administration, or transdermal administration. Such parenteral administration includes, but is not limited to, intravenous injection, subcutaneous injection, and intramuscular injection. In some specific embodiments, the administration route of the tyrosine kinase inhibitor is oral administration.

In some embodiments, the administration route of the anti-HER3 antibody-drug conjugate is oral administration, parenteral administration, or transdermal administration. Such parenteral administration includes, but is not limited to, intravenous injection, subcutaneous injection, and intramuscular injection. In some specific embodiments, the administration route of the anti-HER3 antibody-drug conjugate is intravenous injection administration.

In some embodiments, the administration order of the anti-HER3 antibody-drug conjugate in combination with the tyrosine kinase inhibitor is the anti-HER3 antibody-drug conjugate, followed by the tyrosine kinase inhibitor, with a required administration interval between the two agents (e.g., no more than 1 h, no more than 2 h, etc.).

In some embodiments, the administration order of the anti-HER3 antibody-drug conjugate in combination with the tyrosine kinase inhibitor is the tyrosine kinase inhibitor, followed by the anti-HER3 antibody-drug conjugate, with a required administration interval between the two agents (e.g., no more than 1 h, no more than 2 h, etc.).

## Brief Description of the Drawings

Figure 1 shows the Kaplan-Meier curve for median progression-free survival (PFS) in patients treated with ADC-1.

## Terms

In order that the present invention can be more readily understood, certain technical and scientific terms are specifically defined below. Unless otherwise explicitly defined herein, all other technical and scientific terms used herein have the meanings commonly understood by those of ordinary skill in the art to which the present invention belongs.

Unless the context clearly requires otherwise, throughout the description and the claims, the words "comprising", "having", "including", and the like are to be understood in an inclusive sense, rather than an exclusive or exhaustive sense; that is to say, in the sense of "including but not limited to".

The term "optional" or "optionally" means that the subsequently described event or circumstance may but need not occur, and that the description includes instances where the event or circumstance occurs and instances in which it

does not.

The term "about" or "approximately" refers to a value that is within an acceptable error range for the particular value as determined by those of ordinary skill in the art, which will depend in part on how the value is measured or determined (i.e., the limitations of the measurement system). For example, "about" may mean within 1 or more than 1 standard deviation. Alternatively, "about" or "substantially comprising" may mean a range of up to 20%, such as varying between 1% to 15%, between 1% to 10%, between 1% to 5%, between 0.5% to 5%, or between 0.5% to 1%. In the present invention, each instance where the term "about" precedes a number or a numerical range also includes embodiments of the given number. Unless otherwise stated, when a particular value appears in the present application and claims, the meaning of "about" or "substantially comprising" should be assumed to be within an acceptable error range for that particular value.

The term "and/or", such as "X and/or Y", should be understood to mean "X and Y" or "X or Y", and should be used to provide explicit support for both meanings or either meaning.

In some embodiments, the present invention provides the following definitions.

The term "antibody-drug conjugate (ADC)" refers to a conjugate in which an antibody or antibody fragment is linked via a stable chemical linker compound to a bioactive cytotoxin or a small-molecule drug with cell killing activity. This conjugate takes full advantage of the antibody's specificity of binding to antigens that are specifically or highly expressed on tumor cells and the high potency of cytotoxins, while avoiding toxic side effects on normal cells. Compared with conventional chemotherapeutic drugs in the past, antibody-drug conjugates may specifically bind to tumor cells and reduce the adverse effects on normal cells.

If an antibody-drug conjugate does not show significant chemical changes, the antibody "retains its chemical stability" in the pharmaceutical formulation. Chemical stability may be assessed by detecting and quantifying chemically altered forms of the protein. Degradation processes that commonly alter the chemical structure of proteins include hydrolysis or truncation (evaluated by methods such as size-exclusion chromatography and CE-SDS), oxidation (evaluated by methods such as peptide mapping combined with mass spectrometry or MALDI/TOF/MS), deamidation (evaluated by methods such as ion-exchange chromatography, capillary isoelectric focusing, peptide mapping, and measurement of isoaspartic acid formation), and isomerization (evaluated by methods such as measurement of isoaspartic acid content and peptide mapping).

If the biological activity of an antibody-drug conjugate at a given time is within a predetermined range of its biological activity as exhibited at the time of preparation of the pharmaceutical formulation, the antibody-drug conjugate "retains its biological activity" in the pharmaceutical formulation.

The three-letter and single-letter codes for amino acids used in the present invention are described in J. biol. chem, 243, p 3558 (1968).

As used herein, the term "antibody" is used in the broadest sense, and encompasses various antibody structures, including but not limited to monoclonal antibodies, polyclonal antibodies, monospecific antibodies, multispecific antibodies (e.g., bispecific antibodies), full-length antibodies, and antibody fragments (or antigen-binding fragments or antigen-binding portions), as long as they exhibit the desired antigen-binding activity. The term antibody may refer to an immunoglobulin, which is a tetrapeptide chain structure composed of two identical heavy chains and two identical light chains linked via interchain disulfide bonds. The immunoglobulin heavy chain constant regions have different amino acid compositions and sequence arrangements, and therefore have different antigenicity. Accordingly, immunoglobulins may be classified into five classes, or referred to as the isotypes of immunoglobulins, namely IgM, IgD, IgG, IgA, and IgE, whose corresponding heavy chains are the mu chain, delta chain, gamma chain, alpha chain, and epsilon chain, respectively. Ig of the same class may be further classified into different subclasses based on differences in the amino acid composition of the hinge regions and the number and positions of the heavy chain disulfide bonds, for example, IgG may be divided into IgG1, IgG2, IgG3, and IgG4. Light chains are classified into kappa chains or lambda chains based on differences in the constant regions. Each of the five classes of Ig may have kappa chains or lambda chains. In the antibody heavy chains and light chains, the sequences of about 110 amino acids near the N-termini vary greatly, and are referred to as the variable regions (V regions); and the remaining amino acid sequences near the C-termini are relatively stable, and are referred to as the constant regions (C regions). The variable region comprises three hypervariable regions (HVRs) and four framework regions (FRs) with relatively conserved sequences. The three hypervariable regions determine the specificity of the antibody, and are also referred to as complementarity determining regions (CDRs). Each light chain variable region (VL) and heavy chain variable region (VH) consists of three CDR regions and four FR regions, arranged in the following order from the amino-terminus to the carboxyl-terminus: FR1, CDR1, FR2, CDR2, FR3, CDR3, FR4. The three CDR regions of the light chain refer to LCDR1, LCDR2, and LCDR3; and the three CDR regions of the heavy chain refer to HCDR1, HCDR2, and HCDR3.

For the determination or definition of CDRs, definitive delineation of CDRs and identification of residues comprising the binding site of an antibody may be accomplished by resolving the structure of the antibody and/or resolving the structure of the antibody-ligand complex. This may be achieved by any of a variety of techniques known to those skilled in the art, such as X-ray crystallography. A variety of analytical methods may be used to identify CDRs,

including but not limited to the Kabat numbering system, the Chothia numbering system, the AbM numbering system, the IMGT numbering system, contact definition, and conformational definition.

The amino acid sequence boundaries of CDRs may be determined by various well-known schemes, such as the "Kabat" numbering scheme (see Kabat et al. (1991), "Sequences of Proteins of Immunological Interest", 5th edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), the ImMunoGenTics (IMGT) numbering scheme (see Lefranc, M.P. et al., Dev. Comp. Immunol., 27, 55-77 (2003); Front Immunol. 2018 Oct 16; 9: 2278), and the like.

The term "antigen-binding fragment" or "functional fragment" or "antigen-binding portion" refers to one or more fragments of an intact antibody that retain the ability to specifically bind to an antigen. It has been shown that fragments of full-length antibodies may be utilized for the antigen-binding function of the antibodies. Examples of exemplary binding fragments encompassed within the term "antigen-binding fragment" include (i) a Fab fragment, a monovalent fragment consisting of the VL, VH, CL, and CH1 domains; (ii) an $F(ab')_2$ fragment, a bivalent fragment comprising two Fab fragments linked by a disulfide bridge on the hinge region; (iii) an Fd fragment consisting of the VH and CH1 domains; (iv) an Fv fragment consisting of the VH and VL domains of a single arm of an antibody; (v) a dsFv, a stable antigen-binding fragment formed from the VH and VL via an interchain disulfide bond; (vi) an scFv; and (vii) diabodies, bispecific antibodies, and multispecific antibodies comprising fragments such as scFv, dsFv, and Fab.

The terms "specific binding", "selective binding", "selectively binds", and "specifically binds" refer to the binding of an antibody to an epitope on a predetermined antigen. Typically, the antibody binds with an affinity (KD) of approximately less than $10^{-8}$ M, such as approximately less than $10^{-9}$ M, $10^{-10}$ M, $10^{-11}$ M, or lower.

The term "KD" refers to the equilibrium dissociation constant of a particular antibody-antigen interaction. Typically, the antibody of the present invention binds to IL-5 with an equilibrium dissociation constant (KD) of approximately less than $10^{-7}$ M, such as approximately less than $10^{-8}$ M, $10^{-9}$ M, $10^{-10}$ M or lower, e.g., as determined by surface plasmon resonance (SPR) technology in a BIACORE instrument.

The term "homology" or "identity" refers to sequence similarity between two polynucleotide sequences or between two polypeptides. When a position in both of the two compared sequences is occupied by the same base or amino acid monomer subunit, for example, if a position in each of two DNA molecules is occupied by adenine, then the molecules are homologous at that position. The percent homology between two sequences is a function of the number of matching or homologous positions shared by the two sequences divided by the number of positions compared $\times$ 100. For example, if 6 of 10 of the positions in two sequences are matched or homologous when the sequences are optimally aligned, then the two sequences are 60% homologous; and if 95 of 100 of the positions in two sequences are matched or homologous, then the two sequences are 95% homologous. Typically, a comparison is made when two sequences are aligned to give maximum percent homology. For example, the comparison may be performed by a BLAST algorithm wherein the parameters of the algorithm are selected to give the largest match between the respective sequences over the entire length of the respective reference sequences. The following references relate to the BLAST algorithms commonly used for sequence analysis: Altschul, S.F. et al., (1990) J. Mol. Biol. 215: 403-410; Gish, W. et al., (1993) Nature Genet. 3: 266-272; Madden, T.L. et al., (1996) Meth. Enzymol. 266: 131-141; Altschul, S.F. et al., (1997) Nucleic Acids Res. 25: 3389-3402; and Zhang, J. et al., (1997) Genome Res. 7: 649-656. Other conventional BLAST algorithms such as those provided by NCBI BLAST are also well known to those skilled in the art.

When applied to an animal, human, experimental subject, cell, tissue, organ, or biological fluid, "administering" and "treating" refer to the contact of an exogenous drug, therapeutic agent, diagnostic agent, or composition with the animal, human, subject, cell, tissue, organ, or biological fluid. The terms "administering" and "treating" may refer to, e.g., therapeutic, pharmacokinetic, diagnostic, research, and experimental methods. Treatment of a cell includes the contact of a reagent with the cell, as well as the contact of a reagent with a fluid, where the fluid is in contact with the cell. The terms "administering" and "treating" also mean treating, e.g., cells *in vitro* and *ex vivo* by reagents, diagnostic agents, or binding compositions or by another type of cell. The term "treating", when applied to a human, veterinary, or study subject, refers to a therapeutic treatment, prevention or prophylactic measure, research, and diagnostic applications.

The term "treatment" refers to the internal or external administration of a therapeutic agent to a patient, such as a composition comprising any of the binding compounds of the present invention, wherein the patient has one or more disease symptoms, and the therapeutic agent is known to have a therapeutic effect on such symptoms. Typically, the therapeutic agent is administered in an amount effective to alleviate one or more disease symptoms in the treated patient or population, so as to induce the regression of such symptoms or inhibit the progression of such symptoms to any clinically measurable extent. The amount of a therapeutic agent effective to alleviate any particular disease symptom (also known as a "therapeutically effective amount") may vary depending on a variety of factors, such as the patient's disease state, age and body weight, and the ability of the drug to produce the desired efficacy in the patient. Whether a disease symptom has been alleviated may be evaluated by any clinical testing method commonly used by doctors or other health care professionals to evaluate the severity or progression of the symptom. Although the embodiments of the present invention (e.g., treatment methods or articles of manufacture) may not be effective in alleviating every target disease symptom, they

should reduce the target disease symptoms in a statistically significant number of patients, as determined by any statistical test method known in the art, such as the Student's t-test, Chi-square test, Mann-Whitney U test, Kruskal-Wallis test (H test), Jonckheere-Terpstra test, and Wilcoxon test.

The term "effective amount" includes an amount sufficient to ameliorate or prevent a symptom or condition of a medical disease. The term effective amount also means an amount sufficient to allow or facilitate diagnosis. The effective amount for a particular patient or veterinary subject may vary depending on factors such as the condition to be treated, the patient's general health, the method, route and dose of administration, and the severity of side effects. An effective amount may be the maximum dose or administration regimen that avoids significant side effects or toxic effects.

The term "subject" or "patient" means a mammal, particularly a primate, and especially a human.

The term "combination" as referred to in the present invention is an administration mode that means administering at least one dose of an anti-HER3 antibody-drug conjugate and at least one dose of a tyrosine kinase inhibitor within a certain time period, in which both drugs exhibit pharmacological effects. The time period may be within an administration cycle, preferably within 4 weeks, 3 weeks, 2 weeks, or 1 week, or 24 hours, or 12 hours. The anti-HER2 antibody-drug conjugate or a pharmaceutically acceptable salt thereof and the tyrosine kinase inhibitor may be administered simultaneously or sequentially. Such a period includes treatment in which the anti-HER3 antibody-drug conjugate and the tyrosine kinase inhibitor are administered by the same administration route or by different administration routes. The administration mode of the combination as referred to in the present invention is selected from simultaneous administration, independent formulation and co-administration, or independent formulation and sequential administration.

In the anti-HER3 antibody-drug conjugate of the present invention, "n" refers to the average number of cytotoxic drugs loaded per antibody or its antigen-binding fragment in an antibody-drug conjugate molecule, and it may also be expressed as the ratio of the amount of drug to the amount of antibody, which is the average number of drugs per ADC molecule following the conjugation reaction, as determined by hydrophobic interaction chromatography (HIC) combined with mass spectrometry.

The term "pharmaceutical composition" denotes a mixture containing one or more compounds described herein or physiologically/pharmaceutically acceptable salts or prodrugs thereof and other chemical components, such as physiologically/pharmaceutically acceptable carriers and excipients. The pharmaceutical compositions are intended to facilitate administration to living organisms and enhance the absorption of active ingredients to exert their biological activity.

## Detailed Description of Embodiments

The present invention will be further described below in conjunction with examples, but these examples are not intended to limit the scope of the present invention. In the examples of the present invention, experimental methods for which specific conditions are not specified are generally performed under conventional conditions, e.g., referring to Antibodies: A Laboratory Manual and Molecular Cloning: A Laboratory Manual, Cold Spring Harbor Laboratory Press; or in accordance with the conditions recommended by the manufacturers of the raw materials or commercial products. Reagents for which the specific source is not specified are conventional, commercially available reagents.

## Example 1. Preparation of Anti-HER3 Antibody-Drug Conjugates

The anti-HER3 antibody-drug conjugates are Example 3-1 (n = 4.19), Example 3-2 (n = 2.91), and Example 3-3 (n = 7.27) of WO 2022078425 A1, which is incorporated by reference in its entirety into the present invention, with the structure as follows:

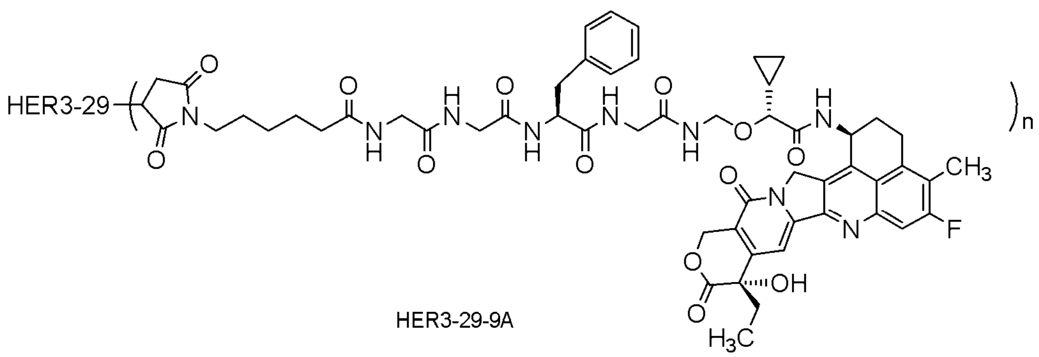

HER3-29-9A

wherein n is 1 to 8, preferably 3 to 5.

The structure of the anti-HER3 antibody-drug conjugates is abbreviated as ADC-1 with the following structural formula:

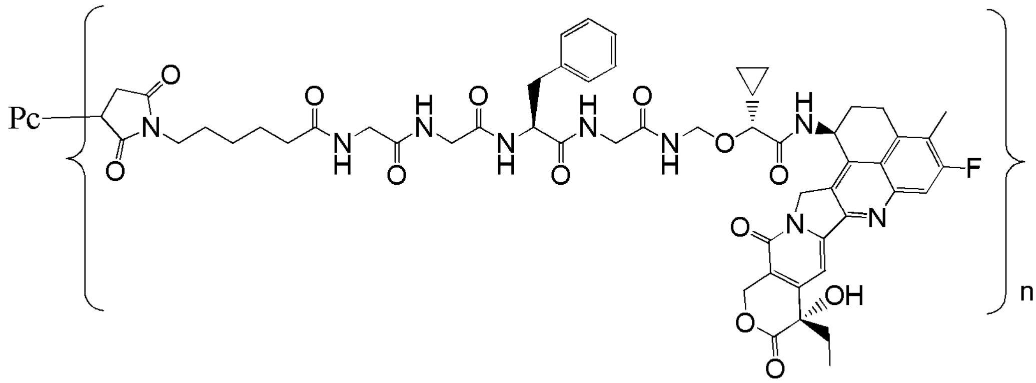

wherein n (DAR value) is about 4.0, equivalent to 4.0 ± 0.4.

The anti-HER3 antibody is derived from antibody HER3-29 in WO 2022078425A1, with its CDR sequences as shown in Table 1. The full-length heavy chain of antibody HER3-29 is as set forth in SEQ ID NO: 9, and the full-length light chain thereof is as set forth in SEQ ID NO: 10.

ADC-1 was formulated into an injectable solution (sterile powder for injection) with a specification of 100 mg per vial. The investigational medical product in a single-use vial was used to prepare the injectable solution. The vial containing 100 mg of the investigational medical product was reconstituted with 5 mL of sterile water for injection to a concentration of 20 mg/mL, and then diluted to the target concentration with 0.9% sodium chloride solution or 5% glucose solution.

## Example 2. Study on the Safety, Tolerability, Pharmacokinetics, and Efficacy of ADC-1 for Injection in Patients with Advanced Solid Tumors

### 1. Investigational medical product

1) ADC-1 prepared in Example 1.

### 2. Enrolled subjects

1) Aged 18 to 75 years (inclusive), either sex;
2) Patients with histologically or cytologically confirmed unresectable locally advanced or metastatic solid tumors, who had experienced recurrence or progression following standard treatment, or had no standard treatment regimen available, or were not applicable to standard treatment at this stage;
3) At least one measurable tumor lesion according to RECIST v1.1 (patients with only non-target lesions were permitted for enrollment during the dose escalation phase);
4) Subjects enrolled in the dose expansion and efficacy expansion phases must provide tumor tissue samples for detection of HER3 expression and the like. The tumor tissue samples were required to be neutral formalin-fixed, paraffin-embedded (FFPE) tissue blocks or at least six unstained tumor tissue sections; and either fresh or archived samples were acceptable, with fresh samples preferred. Subjects unable to provide freshly obtained tissue may provide archived tumor tissue samples collected within 12 months prior to the first study treatment;
5) ECOG performance status score of 0 or 1;
6) Expected survival time of ≥ 12 weeks; and
7) Adequate bone marrow and organ function, with the following test results obtained within 7 days prior to the first study treatment:

blood routine examination (no blood transfusion or hematopoietic stimulating factor therapy within 14 days prior to the examination): absolute neutrophil count (ANC) ≥ 1.5 × $10^9$/L (1,500/$mm^3$), platelet count ≥ 100 × $10^9$/L (100,000/$mm^3$), and hemoglobin (Hgb) ≥ 9.0 g/dL (90 g/L);
hepatic function examination: alanine aminotransferase (ALT) and aspartate aminotransferase (AST) ≤ 3 × ULN

(for patients with confirmed liver metastasis, ALT and AST ≤ 5 × ULN), and total bilirubin ≤ 1.5 × ULN (for patients with confirmed Gilbert's syndrome, total bilirubin ≤ 3 mg/dL);
renal function examination: serum creatinine ≤ 1.5 × ULN or creatinine clearance ≥ 60 mL/min (calculated by the Cockcroft-Gault formula); and
coagulation function examination: prothrombin time and activated partial thromboplastin time ≤ 1.5 × ULN.

### 3. Clinical protocol

An "i3+3" design was adopted for the dose escalation of ADC-1 to determine the maximum tolerated dose (MTD) or maximum administered dose (MAD). Patients with advanced solid tumors were enrolled for dose escalation, with doses escalated sequentially from low to high levels. ADC-1 was administered via intravenous drip once every three weeks (Q3W), and the dose-limiting toxicity (DLT) observation period was 21 days after the first administration. Six doses were preset based on preclinical trial data: 1.5 mg/kg, 3.0 mg/kg, 4.5 mg/kg, 6.0 mg/kg, 7.5 mg/kg, and 9.0 mg/kg.

Primary study endpoints: 1) the maximum tolerated dose (MTD) or maximum administered dose (MAD), providing the recommended phase 2 dose (RP2D) for subsequent studies; and 2) the incidence and severity of adverse events/serious adverse events (graded according to CTCAE v5.0).

### 4. Results

As of the data collection cutoff date, a total of 42 patients were enrolled (83.3% with ECOG 1; 100% at stage IV; 85.7% with non-small cell lung cancer (NSCLC); 31.0% with brain metastasis). The median number of prior systemic treatment lines was 3 (range, 1-11). Among the 36 NSCLC patients, 34 (94.4%) harbored EGFR mutations; and all these patients were resistant to EGFR-TKIs, among whom 85.3% (29/34) had previously received treatment with third-generation EGFR-TKIs. No dose-limiting toxicity (DLT) was observed at the highest dose of 10.5 mg/kg. Grade ≥ 3 treatment-related adverse events (TRAEs) were reported in 13 (31.0%) patients, with the incidence of hematological toxicities ≥ 5%. Among evaluable patients, the objective response rate (ORR) across all tumor types was 25.0% (9/36; 95% CI 12.1-42.2), and the ORR in NSCLC was 30.0% (9/30; 95% CI 14.7-49.4); and the median duration of response (DoR) was all 7.0 months (range, 2.8-8.5). Table 1 lists the tumor responses at different doses. The 6-month progression-free survival (PFS) rate across all tumor types was 46.4% (95% CI 27.0-63.8), and 49.8% (95% CI 28.8-67.8) for NSCLC. After single/multiple administrations at 1.5-9.0 mg/kg, systemic exposures of ADC-1, total antibody, and free toxin increased approximately in a dose-proportional manner; and plasma toxin exposures were low across all dose levels.

Table 2. Dose-related tumor responses

| | 1.5 mg/kg | 3.0 mg/kg | 4.5 mg/kg | 6.0 mg/kg | 7.5 mg/kg | 9.0 mg/kg | 10.5 mg/kg | Total |
|---|---|---|---|---|---|---|---|---|
| All tumors, n | 3 | 3 | 3 | 11 | 3 | 13 | 0 | 36 |
| Objective response rate (ORR*), n (%) | 0 | 1 (33.3) | 1 (33.3) | 3 (27.3) | 1 (33.3) | 3 (23.1) | 0 | 9 (25.0) |
| Disease control rate (DCR), n (%) | 2 (66.7) | 2 (66.7) | 3 (100) | 6 (54.5) | 2 (66.7) | 11 (84.6) | 0 | 26 (72.2) |
| Median duration of response (DoR) (range), months | NE | NE | 7.0 (7.0-7.0) | 5.7 (2.8-8.5) | NE | NE | NE | 7.0 (2.8-8.5) |
| Non-small cell lung cancer (NSCLC)†, n | 3 | 3 | 3 | 8 | 3 | 10 | 0 | 30 |
| Objective response rate (ORR*), n (%) | 0 | 1 (33.3) | 1 (33.3) | 3 (37.5) | 1 (33.3) | 3 (30.0) | 0 | 9 (30.0) |
| Disease control rate (DCR), n (%) | 2 (66.7) | 2 (66.7) | 3 (100) | 5 (62.5) | 2 (66.7) | 9 (90.0) | 0 | 23 (76.7) |

(continued)

| | 1.5 mg/kg | 3.0 mg/kg | 4.5 mg/kg | 6.0 mg/kg | 7.5 mg/kg | 9.0 mg/kg | 10.5 mg/kg | Total |
|---|---|---|---|---|---|---|---|---|
| Median duration of response (DoR) (range), months | NE | NE | 7.0 (7.0-7.0) | 5.7 (2.8-8.5) | NE | NE | NE | 7.0 (2.8-8.5) |

Analyses were performed in patients with post-baseline assessments. * Including unconfirmed responses. † EGFR-WT, n = 2 (PR, n = 1; and SD, n = 1). NE: Not evaluable.

**5. Conclusion**

ADC-1 exhibits tolerable safety and encouraging anti-tumor activity in patients with heavily pretreated advanced solid tumors.

**Example 3. Study on the Safety, Tolerability, Pharmacokinetics, and Efficacy of ADC-1 for Injection in Patients with Advanced Solid Tumors**

**1. Investigational medical product**

1) ADC-1 prepared in Example 1.

**2. Enrolled subjects**

1) Aged 18 to 75 years (inclusive), either sex;
2) Patients with histologically or cytologically confirmed unresectable locally advanced or metastatic solid tumors, who had experienced recurrence or progression following standard treatment, or had no standard treatment regimen available, or were not applicable to standard treatment at this stage;
3) At least one measurable tumor lesion according to RECIST v1.1 (patients with only non-target lesions were permitted for enrollment during the dose escalation phase);
4) Subjects enrolled in the dose expansion and efficacy expansion phases must provide tumor tissue samples for detection of HER3 expression and the like. The tumor tissue samples were required to be neutral formalin-fixed, paraffin-embedded (FFPE) tissue blocks or at least six unstained tumor tissue sections; and either fresh or archived samples were acceptable, with fresh samples preferred. Subjects unable to provide freshly obtained tissue may provide archived tumor tissue samples collected within 12 months prior to the first study treatment;
5) ECOG performance status score of 0 or 1;
6) Expected survival time of ≥ 12 weeks; and
7) Adequate bone marrow and organ function, with the following test results obtained within 7 days prior to the first study treatment:

blood routine examination (no blood transfusion or hematopoietic stimulating factor therapy within 14 days prior to the examination): absolute neutrophil count (ANC) ≥ 1.5 × $10^9$/L (1,500/$mm^3$), platelet count ≥ 100 × $10^9$/L (100,000/$mm^3$), and hemoglobin (Hgb) ≥ 9.0 g/dL (90 g/L);
hepatic function examination: alanine aminotransferase (ALT) and aspartate aminotransferase (AST) ≤ 3 × ULN (for patients with confirmed liver metastasis, ALT and AST ≤ 5 × ULN), and total bilirubin ≤ 1.5 × ULN (for patients with confirmed Gilbert's syndrome, total bilirubin ≤ 3 mg/dL);
renal function examination: serum creatinine ≤ 1.5 × ULN or creatinine clearance ≥ 60 mL/min (calculated by the Cockcroft-Gault formula); and
coagulation function examination: prothrombin time and activated partial thromboplastin time ≤ 1.5 × ULN.

**3. Clinical protocol**

An "i3+3" design was adopted for the dose escalation of ADC-1 to determine the maximum tolerated dose (MTD) or maximum administered dose (MAD). Patients with advanced solid tumors were enrolled for dose escalation, with doses escalated sequentially from low to high levels. ADC-1 was administered via intravenous drip at a dose of 1.5-12.0 mg/kg once every three weeks (Q3W), and the dose-limiting toxicity (DLT) observation period was 21 days after the first administration. One of the indication expansion cohorts evaluated NSCLC patients with epidermal growth factor receptor (EGFR) mutations.

Primary study endpoints: 1) the maximum tolerated dose (MTD) or maximum administered dose (MAD), providing the recommended phase 2 dose (RP2D) for subsequent studies; and 2) the incidence and severity of adverse events/serious adverse events (graded according to CTCAE v5.0).

**4. Results**

Study results: As of the data collection cutoff date (March 30, 2024), a total of 103 NSCLC patients with EGFR mutations were enrolled and received treatment. The median follow-up duration was 8.6 months. The median number of patients who had previously received systemic treatment was 2 (1-7). All patients had received treatment with epidermal growth factor receptor-TKIs, among whom 88.3% had received treatment with third-generation epidermal growth factor receptor-TKIs; and 64.1% had received chemotherapy, among whom 59.2% had received platinum-based chemotherapy (PBC). Among patients treated at the expanded dose of 9.0 mg/kg (n = 52), in the evaluable group, ORR was 46.9% (23/49; 95% CI 32.5-61.7), and DCR was 93.9% (46/49; 95% CI 83.1-98.7); and the responses induced by ADC-1 were durable (Table 2). Table 2 lists the tumor responses across all dose cohorts. Among all treated patients, the median PFS in the 9.0 mg/kg group was 9.6 months (95% CI: 5.7-12.4), and the median PFS across all dose groups was 6.7 months (95% CI: 4.8-9.7).

Table 3. Tumor responses (evaluable group)

| | 9.0 mg/kg | | All doses | |
|---|---|---|---|---|
| | Overall (n = 49) | Prior PBC (n = 28) | Overall (n = 87) | Prior PBC (n = 47) |
| ORR*, % (95% CI) | 46.9% (32.5-61.7) | 50.0% (30.7-69.4) | 39.1% (28.8-50.1) | 38.3% (24.5-53.6) |
| Median DoR† (95% CI), months (mo) | 8.2 (3.1-NR) | 8.2 (3.0-NR) | 7.0 (4.2-8.5) | 8.4 (3.0-NR) |
| Survival probability of DoR at 6 mo†, % (95% CI) | 67.7% (41.6-84.0) | 72.7% (37.1-90.3) | 64.9% (42.8-80.3) | 72.2% (41.7-88.6) |
| DCR, % (95% CI) | 93.9% (83.1-98.7) | 96.4% (81.7-99.9) | 86.2% (77.2-92.7) | 85.1% (71.7-93.8) |
| * Including unconfirmed responses (n = 3). † Kaplan-Meier method.<br>DoR is duration of response. | | | | |

**5. Conclusion**

For NSCLC patients with EGFR mutations who had progressed after treatment with EGFR-TKIs, ADC-1 exhibits encouraging anti-tumor activity and tolerable safety.

**Example 4. Study on the Safety, Tolerability, Pharmacokinetics, and Efficacy of ADC-1 for Injection in Patients with Advanced Solid Tumors**

**1. Investigational medical product**

1) ADC-1 prepared in Example 1.

## 2. Key inclusion criteria

Recurrent/refractory advanced solid tumors (no standard treatment applicable);
ECOG performance status score of 0 or 1; and
Measurable lesions according to RECIST v1.1.

## 3. Patient baseline characteristics

As of the data cutoff date (July 25, 2024), a total of 130 NSCLC patients with EGFR mutations were enrolled and received treatment, among whom 97 patients were treated with ADC-1 at 7.5-9.0 mg/kg. The overall median follow-up duration was 7.2 months.

All patients had previously received treatment with EGFR-TKIs, among whom 89.2% had received third-generation EGFR-TKIs; and 70.0% of patients had received chemotherapy, among whom 64.6% had received platinum-based chemotherapy (see Table 4).

Table 4. Baseline characteristics

| | **7.5 mg/kg (N = 12)** | **8.0 mg/kg (N = 33)** | **9.0 mg/kg (N = 52)** | **7.5-9.0 mg/kg (N = 97)** | **All patients (N = 130)** |
|---|---|---|---|---|---|
| Median age (range), years | 58.5 (49-69) | 60 (39-70) | 58 (37-69) | 59 (37-70) | 58 (33-70) |
| Female | 9 (75.0) | 22 (66.7) | 34 (65.4) | 65 (67.0) | 82 (63.1) |
| ECOG PS 1 | 12 (100.0) | 32 (97.0) | 49 (94.2) | 93 (95.9) | 124 (95.4) |
| Smoking history | | | | | |
| Never smoker | 10 (83.3) | 28 (84.8) | 44 (84.6) | 82 (84.5) | 106 (81.5) |
| Current/former smoker | 2 (16.7) | 5 (15.2) | 8 (15.4) | 15 (15.5) | 24 (18.5) |
| Stage IV at enrollment | 12 (100.0) | 33 (100.0) | 51 (98.1) | 96 (99.0) | 129 (99.2) |
| Brain metastasis | 4 (33.3) | 8 (24.2) | 11 (21.2) | 23 (23.7) | 31 (23.8) |
| Liver metastasis | 4 (33.3) | 6 (18.2) | 11 (21.2) | 21 (21.6) | 27 (20.8) |
| EGFR activating mutations | | | | | |
| Ex19del | 10 (83.3) | 18 (54.5) | 31 (59.6) | 59 (60.8) | 73 (56.2) |
| L858R | 2 (16.7) | 11 (33.3) | 19 (36.5) | 32 (33.0) | 50 (38.5) |
| G719X | 0 | 1 (3.0) | 1 (1.9) | 2 (2.1) | 3 (2.3) |
| L861Q | 0 | 1 (3.0) | 1 (1.9) | 2 (2.1) | 2 (1.5) |
| Median number of prior systemic treatment lines (range) | 2 (1-8) | 2 (1-6) | 2 (1-6) | 2 (1-8) | 2 (1-8) |
| Prior treatment | | | | | |
| EGFR-TKI | 12 (100.0) | 33 (100.0) | 52 (100.0) | 97 (100.0) | 130 (100.0) |
| Third-generation EGFR-TKI | 11 (91.7) | 31 (93.9) | 44 (84.6) | 86 (88.7) | 116 (89.2) |
| Platinum-based chemotherapy (PBC) | 8 (66.7) | 30 (90.9) | 30 (57.7) | 68 (70.1) | 84 (64.6) |
| Immunotherapy | 2 (16.7) | 6 (18.2) | 12 (23.1) | 20 (20.6) | 23 (17.7) |

## 4. Safety and efficacy data

Among patients treated with ADC-1 at 7.5-9.0 mg/kg, in the evaluable population, the objective response rate (ORR) was 40.4% (38/94; 95% CI 30.4%-51.0%), and the disease control rate (DCR) was 93.6% (88/94; 95% CI 86.6%-97.6%); and the responses induced by ADC-1 were durable (see Table 5 and Figure 2). The tumor responses

across all dose groups (7.5-9.0 mg/kg and all dose groups) are as shown in Table 5.

In the full analysis set, the median progression-free survival (PFS) was 9.6 months (95% CI 5.7-12.3) for patients treated at 7.5-9.0 mg/kg, and the median PFS across all dose groups was 6.9 months (95% CI 5.4-9.7) (see Figure 1).

Among patients treated with ADC-1 at 7.5-9.0 mg/kg, grade ≥ 3 treatment-related adverse events (TRAEs) were reported in 60.8% of patients; and all events with an incidence of ≥ 5% were hematological toxicities. Per the investigator's assessment, 7.2% of patients developed treatment-related interstitial lung disease, among whom 2.1% experienced grade ≥ 3 events.

Table 5. Tumor responses

| | **7.5 mg/kg (N = 12)** | **8.0 mg/kg (N = 33)** | **9.0 mg/kg (N = 49)** | **7.5-9.0 mg/kg (N = 94)** | **All patients (N = 126)** |
|---|---|---|---|---|---|
| Best overall response (unconfirmed*), n (%) | | | | | |
| CR | 0 | 0 | 0 | 0 | 0 |
| PR | 4 (33.3) | 11 (33.3) | 23 (46.9) | 38 (40.4) | 48 (38.1) |
| SD | 7 (58.3) | 20 (60.6) | 23 (46.9) | 50 (53.2) | 62 (49.2) |
| Non-CR/Non-PD | 0 | 0 | 0 | 0 | 2 (1.6) |
| PD | 1 (8.3) | 2 (6.1) | 3 (6.1) | 6 (6.4) | 14 (11.1) |
| ORR* (95% CI), % | 33.3 (9.9-65.1) | 33.3 (18.0-51.8) | 46.9 (32.5-61.7) | 40.4 (30.4-51.0) | 38.1 (29.6-47.2) |
| Median confirmed DoR (95% CI), mo | 12.3 (NR-NR) | NR (NR-NR) | 8.3 (4.2-12.6) | 8.3 (4.2-12.3) | 8.2 (5.6-12.3) |
| DCR (95% CI), % | 91.7 (61.5-99.8) | 93.9 (79.8-99.3) | 93.9 (83.1-98.7) | 93.6 (86.6-97.6) | 88.9 (82.1-93.8) |

* Unconfirmed responses due to the data cutoff date preceding the confirmatory scan. CR, complete response; DCR, disease control rate; DoR, duration of response; NR, not reached; ORR, objective response rate; PD, progressive disease; PR, partial response; and SD, stable disease.

### 5. Conclusion

ADC-1 exhibits encouraging anti-tumor activity and tolerable safety profile in NSCLC patients with EGFR mutations who had progressed after treatment with EGFR-TKIs.

## Example 5. Study on the Safety, Tolerability, and Efficacy of ADC-1 for Injection in Combination with Anti-tumor Therapy in Patients with Advanced Solid Tumors

### 1. Investigational medical products

1) ADC-1 prepared in Example 1; and
2) Almonertinib.

### 2. Enrolled subjects

1) Aged 18 to 75 years (inclusive), either sex;
2) Part A1: subjects with unresectable locally advanced or metastatic non-small cell lung cancers that were histologically or cytologically confirmed, and confirmed to have EGFR mutations (sensitive or non-sensitive mutations) with histological or blood specimens; and Part A2: subjects with unresectable locally advanced or metastatic non-small cell lung cancers that were histologically or cytologically confirmed, and confirmed to have EGFR exon 19 deletion or L858R mutation with histological or blood specimens;

3) Part A1: subjects with EGFR-sensitive mutations, who had experienced radiological progression after treatment with EGFR-TKIs, and had previously received ≤ 2 lines of chemotherapy; or subjects with EGFR non-sensitive mutations, who had experienced recurrence or progression following standard treatment, or had no standard treatment regimen available, or were not applicable to standard treatment at this stage; and Part A2: subjects with untreated locally advanced/metastatic NSCLC, who had not received systemic treatment and were judged by the investigator to be suitable for first-line almonertinib therapy; and administration of prior adjuvant or neoadjuvant therapy (e.g., chemotherapy and radiotherapy other than EGFR-TKIs) was permitted;
4) At least one measurable tumor lesion according to RECIST v1.1 (subjects with only non-target lesions were permitted for enrollment during the Phase IB phase);
5) All enrolled subjects must provide tumor tissue samples for detection of HER3 expression and the like (subjects unable to provide tumor tissue samples were permitted for enrollment during the Phase IB phase). The tumor tissue samples were required to be neutral formalin-fixed, paraffin-embedded (FFPE) tissue blocks or at least six unstained tumor tissue sections; and either fresh or archived samples were acceptable, with fresh samples preferred. Subjects unable to provide freshly obtained tissue may provide archived tumor tissue samples collected within 12 months prior to the first study treatment; For subjects unable to provide tumor tissue samples meeting the aforementioned requirements, it was necessary to discuss with the sponsor to determine whether they could be enrolled;
6) ECOG performance status score of 0 or 1;
7) Expected survival time of ≥ 12 weeks; and
8) Adequate bone marrow and organ function, with the following test results obtained within 7 days prior to the first study treatment:

a) blood routine examination (no blood transfusion or hematopoietic stimulating factor therapy within 14 days prior to the examination): absolute neutrophil count (ANC) ≥ 1.5 × $10^9$/L (1,500/$mm^3$), platelet count ≥ 100 × $10^9$/L (100,000/$mm^3$), and hemoglobin (Hgb) ≥ 9.0 g/dL (90 g/L);
b) hepatic function examination (no hepatoprotective agent therapy within 7 days prior to the examination): alanine aminotransferase (ALT) and aspartate aminotransferase (AST) ≤ 3 × ULN (for subjects with confirmed liver metastasis, ALT and AST ≤ 5 × ULN), and total bilirubin ≤ 1.5 × ULN (for subjects with confirmed Gilbert's syndrome, total bilirubin ≤ 3 mg/dL);
c) renal function examination: serum creatinine ≤ 1.5 × ULN or creatinine clearance ≥ 60 mL/min (calculated by the Cockcroft-Gault formula); and
d) coagulation function examination: prothrombin time and activated partial thromboplastin time ≤ 1.5 × ULN.

**3. Administration regimen**

Part A was a clinical trial of ADC-1 in combination with almonertinib for the treatment of EGFR mutation-positive locally advanced/metastatic non-small cell lung cancer, which was divided into Part A1 and Part A2. Part A1 focused on ADC-1 in combination with almonertinib for the treatment of EGFR mutation-positive locally advanced/metastatic NSCLC that had experienced treatment failure with EGFR-TKIs, and Part A2 focused on ADC-1 in combination with almonertinib for the treatment of naïve EGFR mutation-positive locally advanced/metastatic NSCLC. The study consisted of a screening period, a treatment period, and a follow-up period; and the treatment period included the Phase IB and Phase II phases.

Part A1 focused on ADC-1 in combination with almonertinib for the treatment of EGFR mutation-positive locally advanced/metastatic NSCLC that had experienced treatment failure with EGFR-TKIs.

*Phase IB phase:*

1) Part A1, dose group 1: ADC-1 at 9.0 mg/kg once every three weeks (Q3W) in combination with almonertinib at 110 mg once a day (QD);
2) Part A1, dose group 2: ADC-1 at 7.5 mg/kg once every three weeks (Q3W) in combination with almonertinib at 110 mg once a day (QD); and
3) Part A1, dose group 3: ADC-1 at 9.0 mg/kg once every three weeks (Q3W) in combination with almonertinib at 55 mg once a day (QD).

ADC-1 was administered via intravenous drip, Q3W, and almonertinib via oral administration, QD, with a 21-day treatment cycle. The recommended dose of ADC-1 in combination with almonertinib was determined in patients with EGFR mutation-positive locally advanced/metastatic non-small cell lung cancer that had experienced treatment failure

with EGFR-TKIs.

*Phase II phase:*

Arm 1 received treatment with ADC-1 in combination with almonertinib at the recommended dose until progressive disease or other treatment discontinuation criteria were met; and Arm 2 received ADC-1 monotherapy at the Recommended Phase 2 dose (RP2D) until progressive disease or other treatment discontinuation criteria were met. The anti-tumor efficacy of ADC-1 in combination with almonertinib versus ADC-1 monotherapy was evaluated in the treatment of EGFR mutation-positive locally advanced/metastatic NSCLC that had experienced treatment failure with EGFR-TKIs.

Part A2 focused on ADC-1 in combination with almonertinib for the treatment of naïve EGFR mutation-positive locally advanced/metastatic NSCLC.

*Phase IB phase:*

4) Part A2, cohort 1: The patients received treatment with ADC-1 at 6.0 mg/kg Q3W in combination with almonertinib at 110 mg QD until progressive disease or other treatment discontinuation criteria were met; and

5) Part A2, cohort 2: the patients received treatment with ADC-1 at 6.0 mg/kg Q3W in combination with almonertinib at 110 mg QD for six cycles, followed by almonertinib monotherapy at 110 mg QD, until progressive disease or other treatment discontinuation criteria were met.

ADC-1 was administered via intravenous drip at 6.0 mg/kg Q3W, and almonertinib via oral administration at 110 mg QD, with a 21-day treatment cycle. The recommended dose and combined treatment cycle of ADC-1 in combination with almonertinib were determined in patients with naïve EGFR mutation-positive locally advanced/metastatic non-small cell lung cancer.

*Phase II phase:*

Arm 1 received treatment with ADC-1 in combination with almonertinib at the recommended dose; and Arm 2 received almonertinib monotherapy at 110 mg QD. The anti-tumor efficacy of ADC-1 in combination with almonertinib versus almonertinib monotherapy was evaluated in the treatment of naïve EGFR mutation-positive advanced NSCLC.

### 4. Evaluation of results

Efficacy endpoints included ORR, DCR, DoR, PFS, and OS as evaluated by the investigator.

Safety evaluation: Tumor assessment was performed in accordance with the RECIST v1.1 criteria, and all subjects underwent baseline radiological tumor assessment during the screening period. Safety of the investigational medical product was evaluated via recording of adverse events (including serious adverse events), laboratory tests, vital signs and physical examinations, electrocardiography, oxygen saturation, echocardiography/multigated acquisition (MUGA), and the like.

## Example 6. A Randomized, Open-Label, Multicenter Phase III Study of ADC-1 Versus Platinum-Containing Chemotherapy in the Treatment of EGFR-Mutant Advanced or Metastatic Non-Small Cell Lung Cancer that had Experienced Treatment Failure with Epidermal Growth Factor Receptor (EGFR)-Tyrosine Kinase Inhibitor (TKI)

### 1. Investigational medical products

1) ADC-1 prepared in Example 1;
2) Pemetrexed disodium for injection, injection, with a specification of 0.5 g per vial;
3) Carboplatin for injection, injection, with a specification of 0.1 g per vial; and
4) Cisplatin injection, injection, with a specification of 6 ml:30 mg.

## 2. Inclusion criteria

(1) Aged 18 to 75 years (inclusive), either sex;
(2) Subjects with locally advanced or metastatic non-squamous non-small cell lung cancer that was histologically or cytologically confirmed.
Locally advanced or metastatic is defined as being staged as Stage IIIB to Stage IV in accordance with the criteria of the 9th edition of the TNM Staging System of the International Association for the Study of Lung Cancer (IASLC), and no longer amenable to radical surgery or radical chemoradiotherapy;
(3) Subjects with EGFR exon 19 deletion or exon 21 (L858R) substitution mutation as confirmed with histological or blood specimens, with no known other driver gene mutations for which approved targeted drug therapies were available (e.g., ALK fusion, ROS1 fusion, BRAF V600 mutation, etc.);
(4) Subjects with locally advanced or metastatic NSCLC must have received treatment with EGFR TKIs, and subject with the T790M mutation must have received treatment with third-generation EGFR TKIs, and had experienced radiological disease progression during or after the treatment.

- Subjects who had experienced tumor recurrence or metastasis during adjuvant therapy with EGFR TKIs or within 6 months of discontinuation of such therapy could be enrolled;
- subjects with non-metastatic tumors who had previously received neoadjuvant/adjuvant chemotherapy or radical chemoradiotherapy, experienced tumor recurrence or metastasis at least 6 months after completion of the aforementioned treatment, received treatment with EGFR TKIs, and experienced radiological disease progression during or after the treatment could be enrolled; and
- subjects with locally advanced or metastatic NSCLC who were permitted to receive anti-angiogenic therapy or targeted therapy during treatment with EGFR

TKIs (e.g., EGFR/c-MET bispecific antibodies, excluding antibody-drug conjugates) could be enrolled, while administration of other systemic anti-tumor therapies other than the aforementioned treatments was not permitted;
(5) At least one measurable lesion as defined by the RECIST v1.1 criteria;
(6) ECOG performance status score of 0 or 1; and
(7) Expected survival time of ≥ 12 weeks.

## 3. Administration regimen

Experimental group: ADC-1 was administered via intravenous drip at 8.0 mg/kg, and the total dose of ADC-1 was calculated based on the subject's body weight measured before each administration.

Control group: Subjects all received platinum-containing dual-drug chemotherapy, Q3W, with a 21-day treatment cycle. Both drugs were administered on Day 1 of each cycle. Administration order: pemetrexed → carboplatin/cisplatin.

(a) Pemetrexed: intravenous infusion, 500 mg/$m^2$;
(b) Cisplatin: intravenous drip, 75 mg/$m^2$;
body surface area calculation formula: Body surface area ($m^2$) = 0.0061 × height (cm) + 0.0128 × body weight (kg) - 0.1529; and
(c) Carboplatin: intravenous drip, AUC 5 mg/mL/min (Calvert formula);

Carboplatin dose (mg) = (target AUC) × [creatinine clearance (mL/min) + 25]. Creatinine clearance was calculated by the Cockcroft-Gault formula.

## 4. Evaluation of efficacy and safety

Primary endpoint was PFS as assessed by BICR, and secondary endpoints included OS; PFS as assessed by the investigator; and ORR (CR+PR), DoR, and DCR (CR+PR+SD) as assessed by the investigator and BICR.

Subjects were continuously assessed for safety during the study, including the incidence and grade of adverse events (AEs) and serious adverse events (SAEs) (judged in accordance with the NCI-CTCAE v5.0 criteria); changes in vital signs; abnormalities in laboratory test indicators; and the incidence of dose hold, dose reduction, and dose discontinuation due to investigational medical product-related toxicities during the trial.

## Claims

1. Use of an anti-HER3 antibody-drug conjugate in the manufacture of a medicament for treating an EGFR-mutant tumor, **characterized in that** the anti-HER3 antibody-drug conjugate has a structure as shown in the formula below:

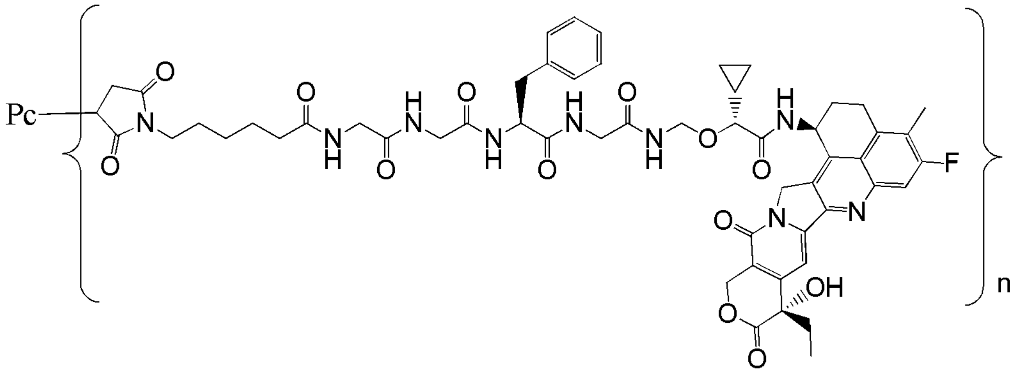

;

wherein: n is 1 to 8, and Pc is an anti-HER3 antibody; and
the EGFR-mutant tumor is preferably EGFR-mutant non-small cell lung cancer.

2. The use according to claim 1, **characterized in that**:

the EGFR-mutant tumor is an EGFR-mutant tumor that has experienced treatment failure with a tyrosine kinase inhibitor (TKI), preferably EGFR-mutant non-small cell lung cancer that has experienced treatment failure with a tyrosine kinase inhibitor (TKI);
the EGFR-mutant tumor is an EGFR-mutant locally advanced or metastatic tumor, preferably EGFR-mutant locally advanced or metastatic non-small cell lung cancer;
or
the EGFR-mutant tumor is an EGFR-mutant locally advanced or metastatic tumor that has experienced treatment failure with a TKI, preferably EGFR-mutant locally advanced or metastatic non-small cell lung cancer that has experienced treatment failure with a TKI.

3. The use according to claim 1 or 2, **characterized in that** the EGFR mutation comprises at least one of the following: exon 19 deletion (Ex19del), L858R mutation, G719X mutation, and L861Q mutation.

4. The use according to any one of claims 1-3, **characterized in that** the EGFR-mutant tumor has brain metastasis, bone metastasis, and/or liver metastasis.

5. The use according to any one of claims 1-4, **characterized in that** the anti-HER3 antibody comprises a heavy chain variable region (VH) comprising HCDR1, HCDR2, and HCDR3 as set forth in SEQ ID NO: 1, SEQ ID NO: 2, and SEQ ID NO: 3, respectively, and a light chain variable region (VL) comprising LCDR1, LCDR2, and LCDR3 as set forth in SEQ ID NO: 4, SEQ ID NO: 5, and SEQ ID NO: 6, respectively; and

preferably, the VH comprises an amino acid sequence as set forth in SEQ ID NO: 7 or an amino acid sequence having at least 80% or 90% identity thereto, and the VL comprises an amino acid sequence as set forth in SEQ ID NO: 8 or an amino acid sequence having at least 80% or 90% identity thereto; and
preferably, the anti-HER3 antibody comprises a heavy chain comprising an amino acid sequence as set forth in SEQ ID NO: 9 or an amino acid sequence having at least 80% or 90% identity thereto, and a light chain comprising an amino acid sequence as set forth in SEQ ID NO: 10 or an amino acid sequence having at least 80% or 90% identity thereto.

6. The use according to any one of claims 1-5, **characterized in that**:

the anti-HER3 antibody-drug conjugate is administered at a dose of about 0.1 mg/kg to about 20 mg/kg, preferably about 1.5 mg/kg to about 12 mg/kg, or about 7.5 mg/kg to about 9.0 mg/kg, and preferably about 1.0 mg/kg, about 1.5 mg/kg, about 3.0 mg/kg, about 4.5 mg/kg, about 6.0 mg/kg, about 6.5 mg/kg, about 7.0 mg/kg, about 7.5

mg/kg, about 8.0 mg/kg, about 8.5 mg/kg, about 9.0 mg/kg, about 10.5 mg/kg, about 12 mg/kg, or about 13.5 mg/kg;
and/or
the anti-HER3 antibody-drug conjugate is administered at a frequency of once a week, once every two weeks, once every three weeks, once every four weeks, once every six weeks, once every eight weeks, once every ten weeks, or once every twelve weeks, preferably about once every three weeks.

**7.** The use according to any one of claims 1-6, **characterized in that** the anti-HER3 antibody-drug conjugate is administered by injection, preferably intravenous injection.

**8.** Use as shown in any one of (1) to (5) below:

(1) use of an anti-HER3 antibody-drug conjugate in combination with a tyrosine kinase inhibitor in the manufacture of a medicament for treating a tumor;
(2) use of a combination of an anti-HER3 antibody-drug conjugate and a tyrosine kinase inhibitor in the manufacture of a medicament for treating a tumor;
(3) use of a pharmaceutical composition in the manufacture of a medicament for treating a tumor, wherein the pharmaceutical composition comprises an anti-HER3 antibody-drug conjugate and a tyrosine kinase inhibitor;
(4) use of an anti-HER3 antibody-drug conjugate for treating a subject with a tumor, wherein the subject is further administered a tyrosine kinase inhibitor; and
(5) use of a tyrosine kinase inhibitor for treating a subject with a tumor, wherein the subject is further administered an anti-HER3 antibody-drug conjugate;
wherein the anti-HER3 antibody-drug conjugate has a structure as shown in the formula below:

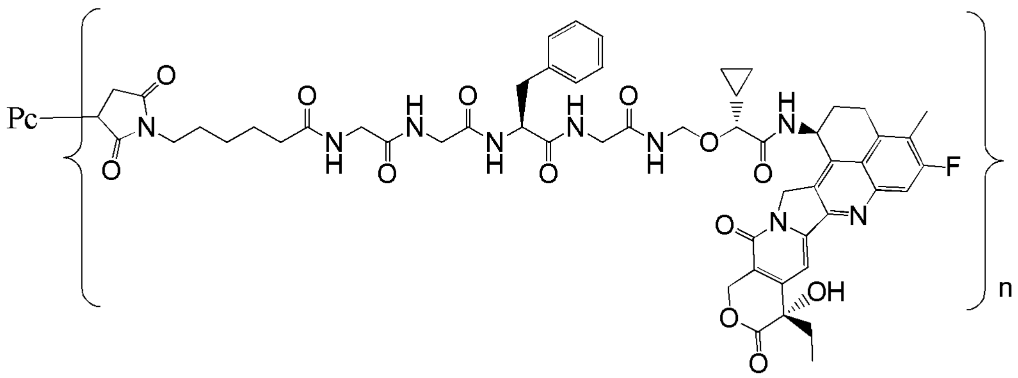
;

wherein: n is 1 to 8, and Pc is an anti-HER3 antibody;
preferably, the tyrosine kinase inhibitor is almonertinib;
preferably, the anti-HER3 antibody comprises a heavy chain variable region (VH) comprising HCDR1, HCDR2, and HCDR3 as set forth in SEQ ID NO: 1, SEQ ID NO: 2, and SEQ ID NO: 3, respectively, and a light chain variable region (VL) comprising LCDR1, LCDR2, and LCDR3 as set forth in SEQ ID NO: 4, SEQ ID NO: 5, and SEQ ID NO: 6, respectively; and more preferably, the VH comprises an amino acid sequence as set forth in SEQ ID NO: 7 or an amino acid sequence having at least 80% or 90% identity thereto, and the VL comprises an amino acid sequence as set forth in SEQ ID NO: 8 or an amino acid sequence having at least 80% or 90% identity thereto; and more preferably, the anti-HER3 antibody comprises a heavy chain comprising an amino acid sequence as set forth in SEQ ID NO: 9 or an amino acid sequence having at least 80% or 90% identity thereto, and a light chain comprising an amino acid sequence as set forth in SEQ ID NO: 10 or an amino acid sequence having at least 80% or 90% identity thereto.

**9.** The use according to claim 8, **characterized in that**: the tumor is an EGFR-mutant tumor, preferably EGFR-mutant non-small cell lung cancer;

preferably, the EGFR-mutant tumor is an EGFR-mutant tumor that has experienced treatment failure with a tyrosine kinase inhibitor (TKI), preferably EGFR-mutant non-small cell lung cancer that has experienced treatment failure with a tyrosine kinase inhibitor (TKI);
the EGFR-mutant tumor is an EGFR-mutant locally advanced or metastatic tumor, preferably EGFR-mutant locally advanced or metastatic non-small cell lung cancer;

or
the EGFR-mutant tumor is an EGFR-mutant locally advanced or metastatic tumor that has experienced treatment failure with a TKI, preferably EGFR-mutant locally advanced or metastatic non-small cell lung cancer that has experienced treatment failure with a TKI.

**10.** The use according to claim 8 or 9, **characterized in that** the EGFR mutation comprises at least one of the following: exon 19 deletion (Ex19del), L858R mutation, G719X mutation, and L861Q mutation.

**11.** The use according to any one of claims 8-10, **characterized in that**:

the anti-HER3 antibody-drug conjugate is administered at a dose of about 0.1 mg/kg to about 20 mg/kg, preferably about 1.5 mg/kg to about 12 mg/kg, or about 7.5 mg/kg to about 9.0 mg/kg, and preferably about 1.0 mg/kg, about 1.5 mg/kg, about 3.0 mg/kg, about 4.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 10.5 mg/kg, about 12 mg/kg, or about 13.5 mg/kg;
the anti-HER3 antibody-drug conjugate is administered at a frequency of once a week, once every two weeks, once every three weeks, once every four weeks, once every six weeks, once every eight weeks, once every ten weeks, or once every twelve weeks, preferably once every three weeks;
and/or
the tyrosine kinase inhibitor is administered at a dose of about 1 mg to about 1000 mg, preferably about 10 mg to about 1000 mg, about 10 mg to about 800 mg, about 10 mg to about 500 mg, about 50 mg to about 500 mg, about 50 mg to about 200 mg, about 50 mg to about 150 mg, or about 55 mg to about 110 mg; and preferably about 50 mg, about 55 mg, about 100 mg, about 110 mg, about 120 mg, about 160 mg, about 200 mg, about 300 mg, about 400 mg, or about 500 mg; and
the tyrosine kinase inhibitor is administered at a frequency of once a day, twice a day, three times a day, once every two days, once every three days, once every four days, once every five days, or once a week, preferably once a day.

**12.** A pharmaceutical composition comprising an anti-HER3 antibody-drug conjugate and a tyrosine kinase inhibitor; wherein

the anti-HER3 antibody-drug conjugate has a structure as shown in the formula below:

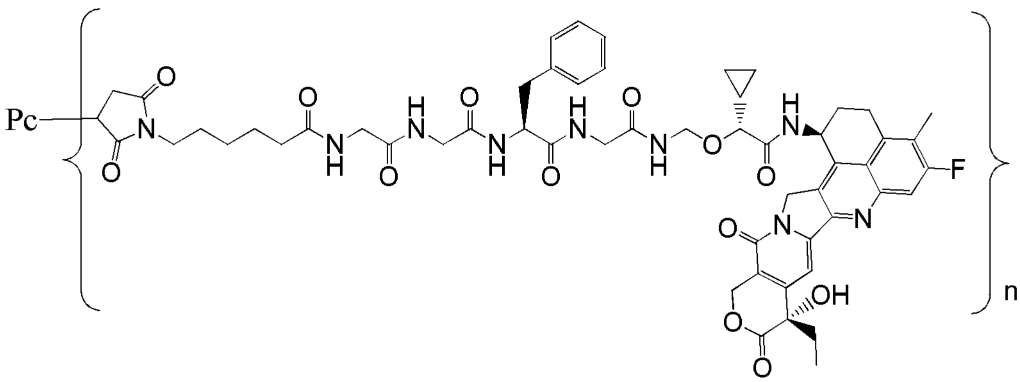

;

wherein: n is 1 to 8, and Pc is an anti-HER3 antibody;
preferably, the tyrosine kinase inhibitor is almonertinib;
preferably, the anti-HER3 antibody comprises a heavy chain variable region (VH) comprising HCDR1, HCDR2, and HCDR3 as set forth in SEQ ID NO: 1, SEQ ID NO: 2, and SEQ ID NO: 3, and a light chain variable region (VL) comprising LCDR1, LCDR2, and LCDR3 as set forth in SEQ ID NO: 4, SEQ ID NO: 5, and SEQ ID NO: 6; and more preferably, the VH comprises an amino acid sequence as set forth in SEQ ID NO: 7 or an amino acid sequence having at least 80% or 90% identity thereto, and the VL comprises an amino acid sequence as set forth in SEQ ID NO: 8 or an amino acid sequence having at least 80% or 90% identity thereto; and
more preferably, the anti-HER3 antibody comprises a heavy chain comprising an amino acid sequence as set forth in SEQ ID NO: 9 or an amino acid sequence having at least 80% or 90% identity thereto, and a light chain comprising an amino acid sequence as set forth in SEQ ID NO: 10 or an amino acid sequence having at least 80%

or 90% identity thereto.

**13.** A method for treating a tumor, comprising administering to a subject in need thereof: (1) an anti-HER3 antibody-drug conjugate; or (2) an anti-HER3 antibody-drug conjugate and a tyrosine kinase inhibitor; wherein

the anti-HER3 antibody-drug conjugate has a structure as shown in the formula below:

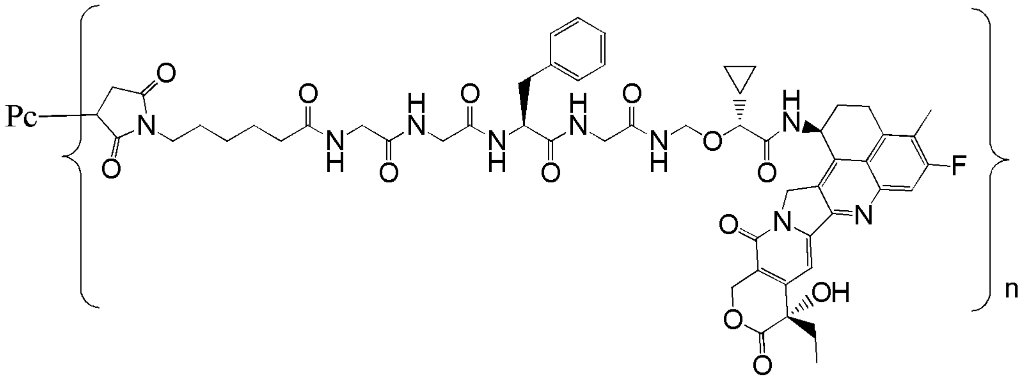

;

wherein: n is 1 to 8, and Pc is an anti-HER3 antibody; and
the anti-HER3 antibody-drug conjugate is administered at a dose of about 0.1 mg/kg to about 20 mg/kg;
preferably, the anti-HER3 antibody-drug conjugate is administered at a dose of about 1.5 mg/kg to about 12 mg/kg, or about 7.5 mg/kg to about 9.0 mg/kg; and preferably about 1.0 mg/kg, about 1.5 mg/kg, about 3.0 mg/kg, about 4.5 mg/kg, about 6.0 mg/kg, about 6.5 mg/kg, about 7.0 mg/kg, about 7.5 mg/kg, about 8.0 mg/kg, about 8.5 mg/kg, about 9.0 mg/kg, about 10.5 mg/kg, about 12 mg/kg, or about 13.5 mg/kg; and/or the anti-HER3 antibody-drug conjugate is administered at a frequency of once a week, once every two weeks, once every three weeks, once every four weeks, once every six weeks, once every eight weeks, once every ten weeks, or once every twelve weeks, preferably once every three weeks; and
preferably, the tyrosine kinase inhibitor is administered at a dose of about 1 mg to about 1000 mg, preferably about 10 mg to about 1000 mg, about 10 mg to about 800 mg, about 10 mg to about 500 mg, about 50 mg to about 500 mg, about 50 mg to about 200 mg, about 50 mg to about 150 mg, or about 55 mg to about 110 mg; and preferably about 50 mg, about 55 mg, about 100 mg, about 110 mg, about 120 mg, about 160 mg, about 200 mg, about 300 mg, about 400 mg, or about 500 mg; and/or the tyrosine kinase inhibitor is administered at a frequency of once a day, twice a day, three times a day, once every two days, once every three days, once every four days, once every five days, or once a week, preferably once a day; and
preferably, the tyrosine kinase inhibitor is almonertinib.

**14.** The method according to claim 13, **characterized in that** the anti-HER3 antibody comprises a heavy chain variable region (VH) comprising HCDR1, HCDR2, and HCDR3 as set forth in SEQ ID NO: 1, SEQ ID NO: 2, and SEQ ID NO: 3, and a light chain variable region (VL) comprising LCDR1, LCDR2, and LCDR3 as set forth in SEQ ID NO: 4, SEQ ID NO: 5, and SEQ ID NO: 6; and more preferably, the VH comprises an amino acid sequence as set forth in SEQ ID NO: 7 or an amino acid sequence having at least 80% or 90% identity thereto, and the VL comprises an amino acid sequence as set forth in SEQ ID NO: 8 or an amino acid sequence having at least 80% or 90% identity thereto; and
more preferably, the anti-HER3 antibody comprises a heavy chain comprising an amino acid sequence as set forth in SEQ ID NO: 9 or an amino acid sequence having at least 80% or 90% identity thereto, and a light chain comprising an amino acid sequence as set forth in SEQ ID NO: 10 or an amino acid sequence having at least 80% or 90% identity thereto.

**15.** The method according to claim 13 or 14, **characterized in that**: the tumor is an EGFR-mutant tumor, preferably EGFR-mutant non-small cell lung cancer;

preferably, the EGFR-mutant tumor is an EGFR-mutant tumor that has experienced treatment failure with a tyrosine kinase inhibitor (TKI), preferably EGFR-mutant non-small cell lung cancer that has experienced treatment failure with a tyrosine kinase inhibitor (TKI);
the EGFR-mutant tumor is an EGFR-mutant locally advanced or metastatic tumor, preferably EGFR-mutant locally advanced or metastatic non-small cell lung cancer;

or
the EGFR-mutant tumor is an EGFR-mutant locally advanced or metastatic tumor that has experienced treatment failure with a TKI, preferably EGFR-mutant locally advanced or metastatic non-small cell lung cancer that has experienced treatment failure with a TKI.

**16.** The method according to any one of claims 13-15, **characterized in that** the EGFR mutation comprises at least one of the following: exon 19 deletion (Ex19del), L858R mutation, G719X mutation, and L861Q mutation.

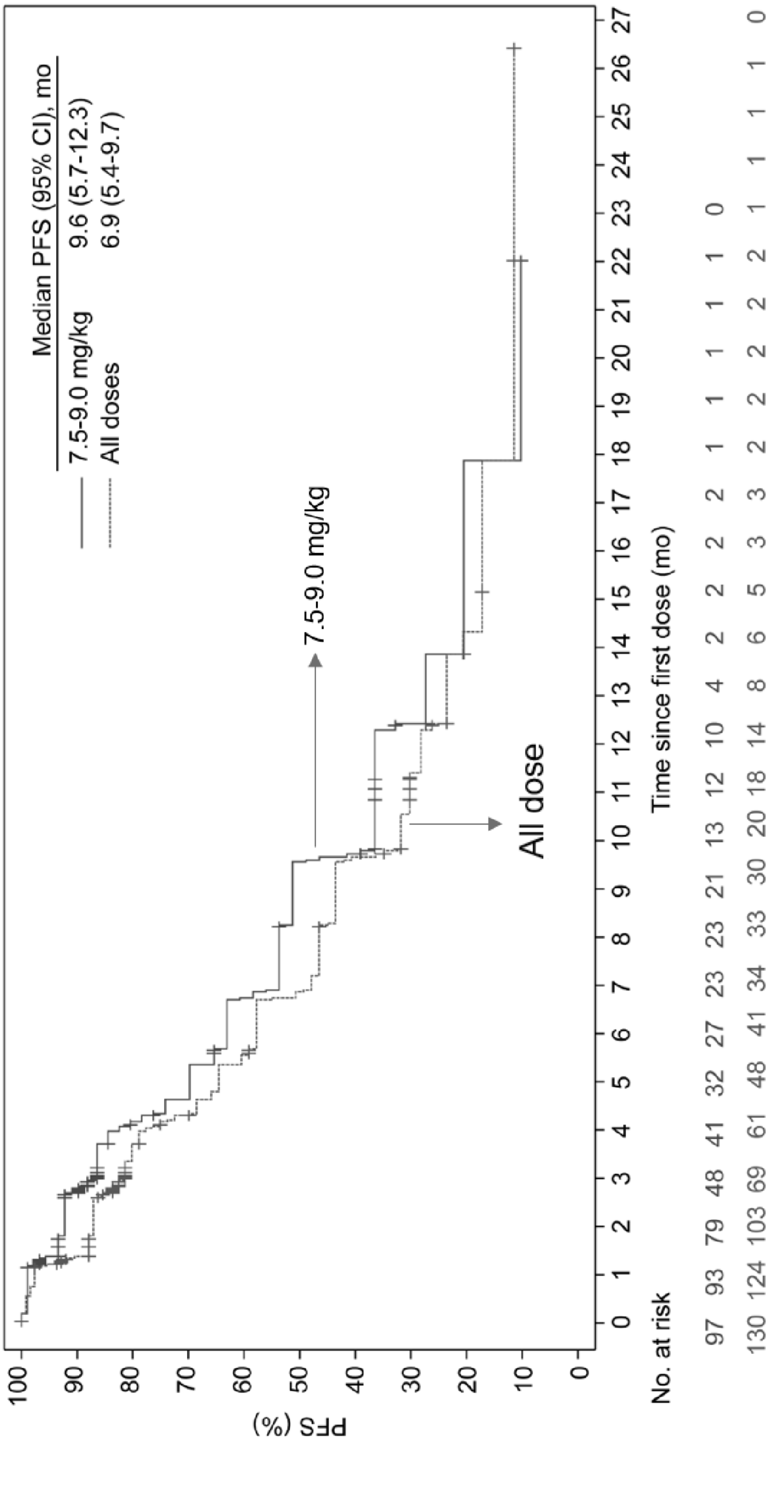

Median PFS (95% CI), mo
7.5-9.0 mg/kg 9.6 (5.7-12.3)
All doses 6.9 (5.4-9.7)
7.5-9.0 mg/kg
All dose
PFS (%)
100 90 80 70 60 50 40 30 20 10 0
0 1 2 3 4 5 6 7 8 9 10 11 12 13 14 15 16 17 18 19 20 21 22 23 24 25 26 27
Time since first dose (mo)
No. at risk
97 93 79 48 41 32 27 23 23 21 13 12 10 4 2 2 2 2 1 1 1 1 1 0
130 124 103 69 61 48 41 34 33 30 20 18 14 8 6 5 3 3 2 2 2 2 2 1 1 1 1 0


*FIG. 1*

## INTERNATIONAL SEARCH REPORT

International application No.
**PCT/CN2024/120021**

**A. CLASSIFICATION OF SUBJECT MATTER**

A61K 47/68(2017.01)i; A61P35/00(2006.01)i; A61K39/395(2006.01)i

According to International Patent Classification (IPC) or to both national classification and IPC

**B. FIELDS SEARCHED**

Minimum documentation searched (classification system followed by classification symbols)

IPC:A61K47; A61K39; A61P35

Documentation searched other than minimum documentation to the extent that such documents are included in the fields searched

Electronic data base consulted during the international search (name of data base and, where practicable, search terms used)

CJFD, CNTXT, ENTXT, ENTXTC, VEN, WPABSC, genebank, 读秀, Duxiu, 百度, Baidu, 必应, Bing, STN: her3, erbB3, her-3, EGFR, HER1, erbB1, 酪氨酸激酶, TKI, 阿美替尼, 依喜替康, 依沙替康, 171335-80-1, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| Y | WO 2022078425 A1 (JIANGSU HENGRUI MEDICINE CO., LTD.; SHANGHAI HENGRUI PHARMACEUTICAL CO., LTD.) 21 April 2022 (2022-04-21)<br>claims 2, 13-14 and 16-18, and description, paragraph 278 | 1-12 |
| Y | CN 110475569 A (KINKI UNIVERSITY et al.) 19 November 2019 (2019-11-19)<br>description, paragraphs 138-199, and embodiments 2-3 | 1-12 |
| Y | WENG, Weining et al. "Antibody-Exatecan Conjugates with a Novel Self-immolative Moiety Overcome Resistance in Colon and Lung Cancer"<br>*Cancer Discovery,* Vol. 13, No. (4), 01 April 2023 (2023-04-01),<br>ISSN: 2159-8274,<br>page 963, left-hand column, paragraph 2 | 1-12 |
| PY | WO 2024114626 A1 (SUZHOU SUNCADIA BIOPHARMACEUTICALS CO., LTD.; SHANGHAI HENGRUI PHARMACEUTICAL CO., LTD.; JIANGSU HENGRUI PHARMACEUTICALS CO., LTD.) 06 June 2024 (2024-06-06)<br>claims 1-12, and description, page 14 | 1-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 December 2024** | **03 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/120021**

**C. DOCUMENTS CONSIDERED TO BE RELEVANT**

| Category* | Citation of document, with indication, where appropriate, of the relevant passages | Relevant to claim No. |
|---|---|---|
| Y | WO 2020063676 A1 (JIANGSU HENGRUI MEDICINE CO., LTD.; SHANGHAI HENGRUI PHARMACEUTICAL CO., LTD.) 02 April 2020 (2020-04-02) claims 26-28 | 1-4, 6-12 |
| A | CN 116712539 A (JIANGSU HENGRUI MEDICINE CO., LTD. et al.) 08 September 2023 (2023-09-08) entire document | 1-12 |
| A | WO 2023061472 A1 (JIANGSU HENGRUI PHARMACEUTICALS CO., LTD.; SHANGHAI SHENGDI PHARMACEUTICAL CO., LTD.) 20 April 2023 (2023-04-20) entire document | 1-12 |

Form PCT/ISA/210 (second sheet) (July 2022)

**INTERNATIONAL SEARCH REPORT**

International application No.
**PCT/CN2024/120021**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No.
**PCT/CN2024/120021**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-16**
because they relate to subject matter not required to be searched by this Authority, namely:

Claims 13-16 set forth a method for treating tumors, wherein the treatment method falls within subject matter for which no search is required by the International Searching Authority as defined in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/120021**

| Patent document cited in search report | Publication date (day/month/year) | Patent family member(s) | Publication date (day/month/year) |
|---|---|---|---|
| WO 2022078425 A1 | 21 April 2022 | CA 3196940 A1 | 21 April 2022 |
| | | CN 116133694 A | 16 May 2023 |
| | | AU 2021360625 A1 | 16 May 2023 |
| | | KR 20230086702 A | 16 May 2023 |
| CN 110475569 A | 19 November 2019 | EP 3590534 A1 | 08 January 2020 |
| | | WO 2018159582 A1 | 07 September 2018 |
| | | TW 201834696 A | 01 October 2018 |
| | | BR 112019015915 A2 | 07 April 2020 |
| | | CA 3053749 A1 | 07 September 2018 |
| | | JP 2022173482 A | 18 November 2022 |
| | | KR 20190120764 A | 24 October 2019 |
| | | JP 7181181 B2 | 30 November 2022 |
| | | KR 20240074000 A | 27 May 2024 |
| | | US 2020061031 A1 | 27 February 2020 |
| | | AU 2018227146 A1 | 29 August 2019 |
| | | SG 11201907050 A1 | 27 September 2019 |
| WO 2024114626 A1 | 06 June 2024 | None | |
| WO 2020063676 A1 | 02 April 2020 | ZA 202102544 B | 25 October 2023 |
| | | JP 2022511351 A | 31 January 2022 |
| | | JP 7558156 B2 | 30 September 2024 |
| | | BR 112021004656 A2 | 01 June 2021 |
| | | EP 3858386 A1 | 04 August 2021 |
| | | EP 3858386 A4 | 12 October 2022 |
| | | KR 20210066833 A | 07 June 2021 |
| | | MX 2021003382 A | 27 May 2021 |
| | | US 2021353764 A1 | 18 November 2021 |
| | | AU 2019349207 A1 | 08 April 2021 |
| | | TW 202027795 A | 01 August 2020 |
| CN 116712539 A | 08 September 2023 | None | |
| WO 2023061472 A1 | 20 April 2023 | TW 202330040 A | 01 August 2023 |
| | | CN 118201613 A | 14 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023112308116
- CN 2024106793284
- WO 2022078425 A1
- WO 2020063676 A1


### Non-patent literature cited in the description


- *J. biol. chem*, 1968, vol. 243, 3558
- **KABAT et al.** Sequences of Proteins of Immunological Interest. Public Health Service, National Institutes of Health, 1991
- **MARTIN, ACR**. *Protein Sequence and Structure Analysis of Antibody Variable Domains [J*, 2001
- **LEFRANC, M.P. et al.** *Dev. Comp. Immunol.*, 2003, vol. 27, 55-77
- *Front Immunol.*, 16 October 2018, vol. 9, 2278
- **ALTSCHUL, S.F. et al.** *J. Mol. Biol.*, 1990, vol. 215, 403-410
- **GISH, W. et al.** *Nature Genet.*, 1993, vol. 3, 266-272
- **MADDEN, T.L. et al.** *Meth. Enzymol.*, 1996, vol. 266, 131-141
- **ALTSCHUL, S.F. et al.** *Nucleic Acids Res.*, 1997, vol. 25, 3389-3402
- **ZHANG, J. et al.** *Genome Res.*, 1997, vol. 7, 649-656
- Antibodies: A Laboratory Manual and Molecular Cloning: A Laboratory Manual. Cold Spring Harbor Laboratory Press